(19) Europäisches Patentamt
European Patent Office
Office européen des brevets

(11) Veröffentlichungsnummer: 0 232 724
B1

(12) # EUROPÄISCHE PATENTSCHRIFT

(45) Veröffentlichungstag der Patentschrift: **02.08.89**

(21) Anmeldenummer: **87100291.1**

(22) Anmeldetag: **12.01.87**

(51) Int. Cl.⁴: **C07D 335/02**, C07D 309/06, C07D 309/22, C07D 307/14, C07D 307/52, C07D 333/22, C07D 213/53, C07D 261/08, C07D 319/06, C07D 319/12, C07D 321/06

(54) **Cyclohexenonderivate, ein Verfahren zu ihrer Herstellung sowie ihre Verwendung als herbizide und das Pflanzenwachstum regulierende Mittel.**

(30) Priorität: **16.01.86 DE 3601066**

(43) Veröffentlichungstag der Anmeldung: **19.08.87 Patentblatt 87/34**

(45) Bekanntmachung des Hinweises auf die Patenterteilung: **02.08.89 Patentblatt 89/31**

(84) Benannte Vertragsstaaten: **AT BE CH DE ES FR GB GR IT LI NL SE**

(56) Entgegenhaltungen: **EP-A- 0 146 837**

(73) Patentinhaber: **BASF Aktiengesellschaft, Carl-Bosch-Strasse 38, D-6700 Ludwigshafen(DE)**

(72) Erfinder: **Zeeh, Bernd, Dr., Queichstrasse 5, D-6703 Limburgerhof(DE)**
Erfinder: **Jahn, Dieter, Dr., Burgunder Weg 8, D-6803 Edingen-Neckarhausen(DE)**
Erfinder: **Keil, Michael, Dr., Fontanestrasse 4, D-6713 Freinsheim(DE)**
Erfinder: **Kolassa, Dieter, Dr., Moltkestrasse 8, D-6700 Ludwigshafen(DE)**
Erfinder: **Würzer, Bruno, Dr., Rüdigerstrasse 13, D-6701 Otterstadt(DE)**
Erfinder: **Meyer, Norbert, Dr., Dossenheimer Weg 22, D-6802 Ladenburg(DE)**
Erfinder: **Rademacher, Wilhelm, Dr., Austrasse 1, D-6703 Limburgerhof(DE)**
Erfinder: **Jung, Johann, Prof. Dr., Hardenburgstrasse 19, D-6703 Limburgerhof(DE)**

EP 0 232 724 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue Cyclohexenonderivate, ein Verfahren zu ihrer Herstellung, herbizide und das Pflanzenwachstum regulierende Mittel, die die neuen Wirkstoffe enthalten und Verfahren zur Bekämpfung von unerwünschtem Pflanzenwachstum sowie zur Regulierung von Pflanzenwachstum.

Die herbizide Wirkung von Cyclohexenonderivaten, die in der Seitenkette in 2-Stellung eine Oximethergruppe enthalten, ist bekannt (DE-A-2 822 304; EP-A-0 099 534; Adv. Pest. Science, Part 2, Pergamon Press, Zürich, 1978; E.H. Geissbühler, Proc. 4th Intern. Congress of Pesticide Chemistry (IUPAC), 1978, 235).

Es ist weiterhin bekannt, daß bestimmte 2-Acyl-3-hydroxycyclohex-2-en-1-one regulierend in das Pflanzenwachstum eingreifen (EP-A-123 001, EP-A-126 713).

Es wurden nun neue Cyclohexenonderivate der FormelI gefunden

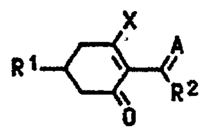

(I),

in der

$R^1$einen 5-bis 7-gliedrigen Heterocyclus mit 1 bis 3 gleichen oder verschiedenen Heteroatomen oder Ringgliedern, ausgewählt aus der Gruppe, umfassend N, O, S, SO und $SO_2$, der gegebenenfalls 1 bis 3 Doppelbindungen und bis zu 3 Substituenten, ausgewählt aus der Gruppe, umfassend $C_1$-$C_6$-Alkyl, $C_2$-$C_6$-Alkenyl, $C_1$-$C_6$-Alkoxy, $C_1$-$C_6$-Alkylthio, $C_1$-$C_4$-Dialkylamino und $C_2$-$C_8$-Alkoxyalkyl enthält, 2-Ethylthiopropyl oder $C_1$-$C_6$-Alkoxycarbonyl,

$R^2$$C_1$-$C_4$-Alkyl,

ASauerstoff oder den Rest $NOR^3$, in dem $R^3$ für $C_1$-$C_4$-Alkyl, $C_3$- und $C_4$-Alkenyl, $C_3$- oder $C_4$-Alkinyl, $C_2$-$C_4$-Halogenalkyl, $C_3$-$C_4$-Halogenalkenyl, $C_2$-$C_4$-Alkoxyalkyl steht, und

X Halogen, Amino, $C_1$-$C_4$-Alkylamino, $C_1$-$C_4$-Dialkylamino, Phenylamino, Pyrrolidino, Piperidino, Morpholino, Hexamethylenimino, $C_1$-$C_4$-Alkylthio, Phenylthio oder ein Azol, ausgewählt aus der Gruppe, umfassend Pyrrol, Pyrazol, Imidazol und 1,2,4-Triazol, das über ein Stickstoffatom an den Cyclohexanring gebunden ist, bedeuten.

Cyclohexenonderivate der Formel I, in der A für den Rest $NOR^3$ steht, besitzen eine vorteilhafte herbizide Wirksamkeit gegen Arten aus der Familie der Gräser (Gramineen).

Diejenigen Cyclohexenonderivate der Formel I, in denen A für Sauerstoff steht, zeichnen sich durch vorteilhafte wachstumsregulatorische Eigenschaften aus.

$R^1$ in Formel I bedeutet beispielsweise Tetrahydropyran-2-yl, Tetrahydropyran-3-yl, 6-Methoxy-tetrahydropyran-2-yl, 6-Methoxy-tetrahydropyran-3-yl, Tetrahydropyran-4-yl, 4-Methyl-tetrahydropyran-3-yl, 3-Methyltetrahydropyran-4-yl, Tetrahydrothiopyran-2-yl, Tetrahydrothiopyran-3-yl, $\Delta^3$ -Dihydrothiopyran-3-yl, Tetrahydrofuran-3-yl, 1-Oxo-tetrahydrothiopran-3-yl, 1,1-Dioxo-tetrahydrothiopyran-3-yl, Tetrahydrothien-3-yl, 2,2-Dimethyltetrahydrothien-3-yl, Pyrid-2-yl, Pyrid-3-yl, 2-Isopropyl-1,3-dioxepan-5-yl, Tetrahydrofuran-2-yl, 1,1-Dioxo-2,2-dimethyl-tetrahydrothien-3-yl, Fur-2-yl, 2-Methylfur-5-yl, Fur-3-yl, Thien-2-yl, Thien-3-yl, 1-Methylpyrrol-2-yl, 1-Methyl-pyrazol-4-yl, 3-Phenyl-isoxazol-5-yl, 4-Methylisothiazol-5-yl, 2-Methylthiazol-5-yl, 2-Dimethylaminothiazol-5-yl, 5,5-Dimethyl-1,3-dioxan-2-yl, Isothiazol-5-yl, 4-Methylisothiazol-5-yl, 2-Ethylthiopropyl oder Ethoxycarbonyl.

$R^2$ in Formel I bedeutet beispielsweise Methyl, Ethyl, Propyl, Isopropyl, Butyl, sec-Butyl, Isobutyl oder tert-Butyl.

Für den Fall, daß in Formel I A für die Gruppe $NOR^3$ steht, bedeutet $R^3$ beispielsweise Methyl, Ethyl, Propyl, Allyl, (E)-But-2-en-1-yl, Propargyl, 2-Chlorethyl, (E)-3-Chlorpropen-1-yl, Methoxymethyl oder 2-Methoxyethyl.

X in Formel I bedeutet beispielsweise Fluor, Chlor, Brom, Amino, Methylamino, Isopropylamino, Dimethylamino, Diethylamino, Phenylamino, Pyrrolidino, Piperidino, Morpholino, Hexamethylenimino, Anilino, Methylthio, Ethylthio, Propylthio, Phenylthio, Pyrrol-1-yl, Pyrazol-1-yl, Imidazol-1-yl oder 1,2,4-Triazol-1-yl.

Bevorzugt sind Cyclohexenonderivate der FormelI, in der $R^1$ Tetrahydropyran-3-yl, Tetrahydropyran-4-yl oder Tetrahydrothiopyran-3-yl, $R^2$ Ethyl oder Propyl und

A Sauerstoff oder den Rest $NOR^3$ bedeuten, wobei $R^3$ für Ethyl, $C_3$-$C_4$-Alkenyl oder $C_3$-$C_4$-Halogenalkenyl steht.

Weiterhin bevorzugt sind die Cyelohexenondreivate gemäss Anspruch 2.

Die neuen Cyclohexenonderivate können vorteilhaft erhalten werden, wenn man eine Verbindung der Formel II

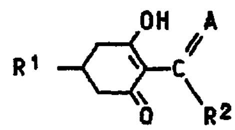

(II),

in der $R^1$, $R^2$ und A die oben genannte Bedeutung besitzen, zunächst mit einem Chlorierungs- oder Bromierungsmittel, gegebenenfalls in Gegenwart einer Base, bei einer Temperatur von -10 bis +120°C, vorzugsweise 20 bis 80°C umsetzt.

Als Chlorierungs- oder Bromierungsmittel verwendet man zweckmäßig anorganische oder organische Säurechloride oder -bromide, beispielsweise Thionylchlorid, Thionylbromid, Phosgen, Phosphoroxychlorid, Phosphoroxybromid, Phosphorpentachlorid, Phosphorpentabromid oder Oxalylchlorid.

Als Basen kommen tertiäre Aminbasen, wie Triethylamin, N,N-Dimethylaminocyclohexan oder Pyridin in Betracht.

Tricarbonylverbindungen und Halogenierungsmittel werden im Molverhältnis 1:1 bis 1:20 eingesetzt.

Für den Fall, daß man in Gegenwart einer Base arbeitet, gibt man von dieser 1 bis 20 Mol, bezogen auf ein Mol Tricarbonylverbindungen, zu.

In manchen Fällen kann es von Vorteil sein, in Gegenwart eines inerten Lösungsmittels, z.B. Benzol, Toluol, Chloroform, Dichlormethan, 1,2-Dichlorethan, Dioxan, Tetrahydrofuran, N,N-Dimethylformamid oder N-Methylpyrrolidon, zu arbeiten.

Die Umsetzung ist nach wenigen Stunden beendet. Das Zielprodukt der Formel I, in der X Chlor oder Brom bedeutet, wird durch Einengen der Reaktionsmischung erhalten.

Diejenigen Verbindungen der Formel I, in denen X eine andere Bedeutung als Chlor oder Brom besitzt, werden durch Umsetzung der jeweiligen Chlor- oder Bromverbindung mit einem Nucleophil der Formel X-H, in der X mit Ausnahme von Chlor und Brom die obengenannte Bedeutung besitzt, bei einer Temperatur von -10 bis +120°C erhalten.

Gegebenenfalls wird diese Umsetzung in Gegenwart einer tertiären Aminbase, z.B. Triethylamin, N,N-Dimethylaminocyclohexan oder Pyridin durchgeführt.

Für den Fall, daß man ein Fluoratom in den Cyclohexanring einbauen will, empfiehlt es sich nicht mit freiem Fluorwasserstoff zu arbeiten, sondern diesen zunächst in ein Fluoridsalz überzuführen, bzw. direkt mit Fluorid zu arbeiten.

Chlor- oder Bromverbindung und Nucleophil (X-H) werden im Molverhältnis 1:1 bis 1:20 eingesetzt. Wen man in Gegenwart einer Base arbeitet, gibt man von dieser 1 bis 20 Mol, bezogen auf die Chlor- oder Bromverbindung, zu.

Die Substitutionsreaktion kann auch in Gegenwart eines inerten Lösungsmittels, beispielsweise Benzol, Toluol, Chloroform, Dichlormethan, 1,2-Dichlorethan, Dioxan, Tetrahydrofuran, N,N-Dimethylformamid oder N-Methylpyrrolidon vorgenommen werden.

Die Umsetzung ist nach wenigen Stunden beendet, danach kann das Reaktionsprodukt durch Einengen der Reaktionslösung, Aufnehmen in Methylenchlorid und Ausschütteln mit verdünnter Natriumcarbonatlösung und Wasser erhalten werden. Nach Entfernen des Lösungsmittels können die so erhaltenen Rohprodukte säulenchromatographisch über Kieselgel gereinigt werden.

Die Verbindungen der Formel II sind bekannt und z.B. in der DE-A-28 22 304, EP-A- 0 066 195, EP-A-123 001 und EP-A-0 131 875 beschrieben.

Die nun folgenden Beispiele sollen die Erfindung näher erläutern.

Beispiel 1

141,2 g (0,5 mol) 2-Butyryl-3-hydroxy-5-(tetrahydrothiopyran-3-yl)-cyclohex-2-en-1-on wurden bei 0 bis 5°C mit 420 ml Oxalylchlorid versetzt, 16 Stunden bei Raumtemperatur gerührt und eingeengt. Das erhaltene Öl wurde mit Methyl-tert-Butylether behandelt und der dabei entstehende Feststoff abgesaugt und getrocknet. Man erhielt 125,9 g (84% Ausbeute) 2-Butyryl-3-chlor-5-(tetrahydrothiopyran-3-yl)-cyclohex-2-en-1-on vom Fp. 97°C (Zers.) (Verbindung Nr.312).

Beispiel 2

10,0 g (31 mmol) 2-(1-Ethoxyiminobutyl-3-hydroxy-5-(tetrahydrothiopyran-3-yl)-cyclohex-2-en-1-on wurden in 50 ml Tetrahydrofuran gelöst und mit 4,7 ml Triethylamin versetzt. Anschließend wurden 2,3 ml Thionylchlorid zugegeben. Nach 3 Stunden wurde der entstandene Niederschlag abgesaugt, mit Tetrahydrofuran nachgewaschen und das produkthaltige Filtrat eingeengt. Man erhielt 3-Chlor-2-(1-ethoxyiminobutyl)-5-(tetrahydrothiopyran-3-yl)-cyclohex-2-en-1-on als Öl (Verbindung Nr. 90).

Beispiel 3

Eine Lösung aus 4,5 ml (50 mmol) Propanthiol, 125 ml Tetrahydrofuran, 7 ml Triethylamin und 15,0 g (50 mmol) 2-Butyryl-3-chlor-5-(tetrahydrothiopyran)-cyclohex-2-en-1-on wurde 4 Stunden unter Rückfluß

erhitzt, vom gebildeten Niederschlag abfiltriert und das Filtrat eingeengt. Der erhaltene Rückstand wurde in 300 ml Dichlormethan aufgenommen, zweimal mit je 200 ml Wasser ausgeschüttelt und über Natriumsulfat getrocknet. Nach Einengen erhielt man 6,6g 2-Butyryl-3-propylthio-5-(tetrahydrothiopyran-3-yl)-cyclohex-2-en-1-on als Öl (Verbindung Nr.323).

Beispiel 4

Zu einer Lösung von 6,8g (0,1mol) Pyrazol in 100 ml N,N-Dimethylformamid wurde eine Lösung von 10,0 g (33 mmol) 2-Butyryl-3-chlor-5-(tetrahydrothiopyran-3-yl)-cyclohex-2-en-1-on in 80 ml N,N-Dimethylformamid getropft. Nach 4 Stunden Rühren wurde eingeengt und der erhaltene Rückstand über Kieselgel chromatographiert. Man erhielt 7,3g 2-Butyryl-3-(pyrazol-1-yl)-5-(tetrahydrothiopyran-3-yl)-cyclohex-2-en-1-on vom Fp. 127°C (Verbindung Nr.325).

Beispiel 5

10,3 g (30 mmol) 3-Chlor-2-(1-ethoxyiminobutyl)-5-(tetrahydrothiopyran-3-yl)-cyclohex-2-en -1-on, 5,1 ml (60 mmol) Isopropylamin und 100 ml Toluol wurden 6 Stunden bei 60°C gerührt. Nach dem Abkühlen wurde dreimal mit je 75 ml Wasser ausgeschüttelt, über Natriumsulfat getrocknet und eingeengt. Der so erhaltene Rückstand wurde über Kieselgel chromatographiert. Man erhielt 3,8g 2-Ethoxyimino-3-isopropylamino-5-(tetrahydrothiopyran-3-yl)-cyclohex-2-en-1-on vom Fp. 104°C (Verbindung Nr. 103).

Die in den nachfolgenden Tabellen 1 und 2 aufgeführten Verbindungen können in analoger Weise erhalten werden.

Tabelle 1

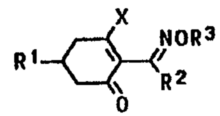

(Ib)

| Verbindung Nr. | $R^1$ | $R^2$ | $R^3$ | X |
|---|---|---|---|---|
| 1 | Tetrahydrofuran-2-yl | Ethyl | $C_2H_5$ | Cl |
| 2 | Tetrahydrofuran-2-yl | Propyl | $C_2H_5$ | Cl |
| 3 | Tetrahydrofuran-3-yl | Ethyl | $C_2H_5$ | Cl |
| 4 | Tetrahydrofuran-3-yl | Propyl | $C_2H_5$ | Cl |
| 5 | Tetrahydrofuran-3-yl | Propyl | $C_2H_5$ | $NH_2$ |
| 6 | Tetrahydrofuran-3-yl | Propyl | $C_2H_5$ | $NHCH_3$ |
| 7 | Tetrahydrofuran-3-yl | Propyl | $C_2H_5$ | 1-Pyrazolyl |
| 8 | Tetrahydrofuran-3-yl | Propyl | $C_2H_5$ | $C_2H_5$ |
| 9 | Tetrahydrothien-3-yl | Ethyl | $C_2H_5$ | Cl |
| 10 | Tetrahydrothien-3-yl | Propyl | $C_2H_5$ | Cl |
| 11 | 2,2-Dimethyl-2,5-dihydrothien-3-yl | Ethyl | $C_2H_5$ | Cl |
| 12 | 2,2-Dimethyl-2,5-dihydrothien-3-yl | Propyl | $C_2H_5$ | Cl |
| 13 | 2-Furyl | Ethyl | $C_2H_5$ | Cl |
| 14 | 2-Furyl | Propyl | $C_2H_5$ | Cl |
| 15 | 2-Furyl | Propyl | $C_2H_5$ | $NH_2$ |
| 16 | 2-Furyl | Propyl | $C_2H_5$ | 1-Pyrrolidinyl |
| 17 | 2-Furyl | Propyl | $C_2H_5$ | N-Morpholino |
| 18 | 2-Furyl | Propyl | $C_2H_5$ | Phenylamino |
| 19 | 2-Furyl | Ethyl | $C_2H_5$ | Cl |

**Tabelle 1** (Fortsetzung)

| Verbindung Nr. | $R^1$ | $R^2$ | $R^3$ | X |
|---|---|---|---|---|
| 20 | 2-Furyl | Propyl | $C_2H_5$ | Cl |
| 21 | 2-Thienyl | Ethyl | $C_2H_5$ | Cl |
| 22 | 2-Thienyl | Propyl | $C_2H_5$ | Cl |
| 23 | 3-Thienyl | Ethyl | $C_2H_5$ | Cl |
| 24 | 3-Thienyl | Propyl | $C_2H_5$ | Cl |
| 25 | 2,5-Dimethyl-thien-3-yl | Ethyl | $C_2H_5$ | Cl |
| 26 | 2,5-Dimethyl-thien-3-yl | Propyl | $C_2H_5$ | Cl |
| 27 | 3-Methyl-isoxazol-5-yl | Ethyl | $C_2H_5$ | Cl |
| 28 | 3-Methyl-isoxazol-5-yl | Propyl | $C_2H_5$ | Cl |
| 29 | 3-Isopropyl-isoxazol-5-yl | Ethyl | $C_2H_5$ | Cl |
| 30 | 3-Isopropyl-isoxazol-5-yl | Propyl | $C_2H_5$ | Cl |
| 31 | 4-Methyl-isothiazol-5-yl | Ethyl | $C_2H_5$ | Cl |
| 32 | 4-Methyl-isothiazol-5-yl | Propyl | $C_2H_5$ | Cl |
| 33 | Isothiazol-4-yl | Ethyl | $C_2H_5$ | Cl |
| 34 | Isothiazol-4-yl | Propyl | $C_2H_5$ | Cl |
| 35 | 2-Methyl-thiazol-4-yl | Ethyl | $C_2H_5$ | Cl |
| 36 | 2-Meth~~x~~yl-thiazol-4-yl | Propyl | $C_2H_5$ | Cl |
| 37 | 2-Methyl-thiazol-5-yl | Ethyl | $C_2H_5$ | Cl |
| 38 | 2-Methyl-thiazol-5-yl | Propyl | $C_2H_5$ | Cl |
| 39 | 2-Phenyl-thiazol-4-yl | Ethyl | $C_2H_5$ | Cl |
| 40 | Tetrahydropyran-2-yl | Ethyl | $C_2H_5$ | Cl |

**Tabelle 1** (Fortsetzung)

| Verbindung Nr. | $R^1$ | $R^2$ | $R^3$ | X |
|---|---|---|---|---|
| 41 | Tetrahydropyran-2-yl | Propyl | $C_2H_5$ | Cl |
| 42 | 6-Methyl-tetrahydropyran-2-yl | Ethyl | $C_2H_5$ | Cl |
| 43 | 6-Methyl-tetrahydropyran-2-yl | Propyl | $C_2H_5$ | Cl |
| 44 | 6-Methoxy-tetrahydropyran-2-yl | Ethyl | $C_2H_5$ | Cl |
| 45 | 6-Methoxy-tetrahydropyran-2-yl | Propyl | $C_2H_5$ | Cl |
| 46 | 6-Ethoxy-tetrahydropyran-2-yl | Ethyl | $C_2H_5$ | Cl |
| 47 | 6-Ethoxy-tetrahydropyran-2-yl | Propyl | $C_2H_5$ | Cl |
| 48 | Tetrahydropyran-3-yl | Methyl | $C_2H_5$ | Cl |
| 49 | Tetrahydropyran-3-yl | Methyl | $CH_2-CH=CH_2$ | Cl |
| 50 | Tetrahydropyran-3-yl | Methyl | $CH_2-CH=CH_2$ | $NH_2$ |
| 51 | Tetrahydropyran-3-yl | Methyl | $CH_2-CH=CH_2$ | 1-Pyrazolyl |
| 52 | Tetrahydropyran-3-yl | Ethyl | $C_2H_5$ | Cl |
| 53 | Tetrahydropyran-3-yl | Propyl | $C_2H_5$ | Cl |
| 54 | Tetrahydropyran-3-yl | Propyl | $C_2H_5$ | $NH_2$ |
| 55 | Tetrahydropyran-3-yl | Propyl | $C_2H_5$ | $NHCH_3$ |
| 56 | Tetrahydropyran-3-yl | Propyl | $C_2H_5$ | $C_2H_5S$ |
| 57 | Tetrahydropyran-3-yl | Propyl | $C_2H_5$ | 1,2,4-Triazol-1-yl |
| 58 | Tetrahydropyran-3-yl | Propyl | $C_2H_5$ | 1-Pyrazolyl |
| 59 | Tetrahydropyran-3-yl | Propyl | $C_2H_5$ | 1-Imidazolyl |
| 60 | Tetrahydropyran-3-yl | Propyl | $C_2H_5$ | N-Piperidyl |
| 61 | Tetrahydropyran-3-yl | Propyl | $C_2H_5$ | N-Morpholino |

Tabelle 1 (Fortsetzung)

| Verbindung Nr. | $R^1$ | $R^2$ | $R^3$ | X |
|---|---|---|---|---|
| 62 | 4-Methyl-tetrahydropyran-3-yl | Ethyl | $C_2H_5$ | Cl |
| 63 | 4-Methyl-tetrahydropyran-3-yl | Propyl | $C_2H_5$ | Cl |
| 64 | 2-Methoxy-tetrahydropyran-3-yl | Ethyl | $C_2H_5$ | Cl |
| 65 | 4-Methyl-tetrahydropyran-3-yl | Propyl | $C_2H_5$ | Cl |
| 66 | Tetrahydropyran-4-yl | Methyl | $C_2H_5$ | Cl |
| 67 | Tetrahydropyran-4-yl | Ethyl | $C_2H_5$ | Cl |
| 68 | Tetrahydropyran-4-yl | Propyl | $C_2H_5$ | Cl |
| 69 | Tetrahydropyran-4-yl | Isopropyl | $C_2H_5$ | Cl |
| 70 | Tetrahydropyran-4-yl | Butyl | $C_2H_5$ | Cl |
| 71 | Tetrahydropyran-4-yl | Ethyl | (E)-$CH_2$-CH=CH-Cl | Cl |
| 72 | Tetrahydropyran-4-yl | Ethyl | (E)-$CH_2$-CH=CH-Cl | $NH_2$ |
| 73 | Tetrahydropyran-4-yl | Ethyl | (E)-$CH_2$-CH=CH-Cl | $NHCH_3$ |
| 74 | Tetrahydropyran-4-yl | Ethyl | (E)-$CH_2$-CH=CH-Cl | $N(CH_3)_2$ |
| 75 | Tetrahydropyran-4-yl | Ethyl | (E)-$CH_2$-CH=CH-Cl | Phenylamino |
| 76 | Tetrahydropyran-4-yl | Ethyl | (E)-$CH_2$-CH=CH-Cl | Isopropylamino |
| 77 | Tetrahydropyran-4-yl | Ethyl | (E)-$CH_2$-CH=CH-Cl | 1-Pyrrolidinyl |
| 78 | Tetrahydropyran-4-yl | Ethyl | (E)-$CH_2$-CH=CH-Cl | $C_2H_5S$ |
| 79 | Tetrahydropyran-4-yl | Ethyl | (E)-$CH_2$-CH=CH-Cl | 1-Pyrazolyl |
| 80 | Tetrahydropyran-4-yl | Ethyl | (E)-$CH_2$-CH=CH-Cl | 1,2,4-Triazol-1-yl |
| 81 | 3-Methyl-tetrahydropyran-4-yl | Ethyl | $C_2H_5$ | Cl |
| 82 | 3-Methyl-tetrahydropyran-4-yl | Propyl | $C_2H_5$ | Cl |

**Tabelle 1** (Fortsetzung)

| Verbindung Nr. | $R^1$ | $R^2$ | $R^3$ | X |
|---|---|---|---|---|
| 83 | $\Delta^3$-Dihydropyran-3-yl | Ethyl | $C_2H_5$ | Cl |
| 84 | $\Delta^3$-Dihydropyran-3-yl | Propyl | $C_2H_5$ | Cl |
| 85 | Tetrahydrothiopyran-3-yl | Methyl | $C_2H_5$ | Cl |
| 86 | Tetrahydrothiopyran-3-yl | Ethyl | $C_2H_5$ | Cl |
| 87 | Tetrahydrothiopyran-3-yl | Isopropyl | $C_2H_5$ | Cl |
| 88 | Tetrahydrothiopyran-3-yl | Butyl | $C_2H_5$ | Cl |
| 89 | Tetrahydrothiopyran-3-yl | Isobutyl | $C_2H_5$ | Cl |
| 90 | Tetrahydrothiopyran-3-yl | Propyl | $C_2H_5$ | Cl |
| 91 | Tetrahydrothiopyran-3-yl | Isobutyl | $C_2H_5$ | F |
| 92 | Tetrahydrothiopyran-3-yl | Propyl | $C_2H_5$ | Br |
| 93 | Tetrahydrothiopyran-3-yl | Propyl | $C_2H_5$ | $NH_2$ |
| 94 | Tetrahydrothiopyran-3-yl | Propyl | $C_2H_5$ | $NHCH_3$ |
| 95 | Tetrahydrothiopyran-3-yl | Propyl | $C_2H_5$ | $N(CH_3)_2$ |
| 96 | Tetrahydrothiopyran-3-yl | Propyl | $C_2H_5$ | $CH_3S$ |
| 97 | Tetrahydrothiopyran-3-yl | Propyl | $C_2H_5$ | $n-C_3H_7S$ |
| 98 | Tetrahydrothiopyran-3-yl | Propyl | $C_2H_5$ | Phenylthio |
| 99 | Tetrahydrothiopyran-3-yl | Propyl | $C_2H_5$ | 1-Pyrazolyl |
| 100 | Tetrahydrothiopyran-3-yl | Propyl | $C_2H_5$ | 1,2,4-Triazol-1-yl |
| 101 | Tetrahydrothiopyran-3-yl | Propyl | $C_2H_5$ | 1-Pyrrolidinyl |
| 102 | Tetrahydrothiopyran-3-yl | Propyl | $C_2H_5$ | N-Morpholino |
| 103 | Tetrahydrothiopyran-3-yl | Propyl | $C_2H_5$ | Isopropylamino |

**Tabelle 1** (Fortsetzung)

| Verbindung Nr. | $R^1$ | $R^2$ | $R^3$ | X |
|---|---|---|---|---|
| 104 | Tetrahydrothiopyran-3-yl | Propyl | $C_2H_5$ | Phenylamino |
| 105 | Tetrahydrothiopyran-3-yl | Propyl | $C_2H_5$ | 1-Imidazolyl |
| 106 | Tetrahydrothiopyran-3-yl | Propyl | $CH_2-CH=CH_2$ | Cl |
| 107 | Tetrahydrothiopyran-3-yl | Propyl | $CH_2-CH=CH_2$ | F |
| 108 | Tetrahydrothiopyran-3-yl | Propyl | $CH_2-CH=CH_2$ | Br |
| 109 | Tetrahydrothiopyran-3-yl | Propyl | $CH_2-CH=CH_2$ | $NH_2$ |
| 110 | Tetrahydrothiopyran-3-yl | Propyl | $CH_2-CH=CH_2$ | $NHCH_3$ |
| 111 | Tetrahydrothiopyran-3-yl | Propyl | $CH_2-CH=CH_2$ | $N(CH_3)_2$ |
| 112 | Tetrahydrothiopyran-3-yl | Propyl | $CH_2-CH=CH_2$ | $CH_3S$ |
| 113 | Tetrahydrothiopyran-3-yl | Propyl | $CH_2-CH=CH_2$ | $n-C_3H_7S$ |
| 114 | Tetrahydrothiopyran-3-yl | Propyl | $CH_2-CH=CH_2$ | Phenylthio |
| 115 | Tetrahydrothiopyran-3-yl | Propyl | $CH_2-CH=CH_2$ | 1-Pyrazolyl |
| 116 | Tetrahydrothiopyran-3-yl | Propyl | $CH_2-CH=CH_2$ | 1,2,4-Triazol-1-yl |
| 117 | Tetrahydrothiopyran-3-yl | Propyl | $CH_2-CH=CH_2$ | 1-Pyrrolidinyl |
| 118 | Tetrahydrothiopyran-3-yl | Propyl | $CH_2-CH=CH_2$ | N-Morpholino |
| 119 | Tetrahydrothiopyran-3-yl | Propyl | $CH_2-CH=CH_2$ | Isopropylamino |
| 120 | Tetrahydrothiopyran-3-yl | Propyl | $CH_2-CH=CH_2$ | Phenylamino |
| 121 | Tetrahydrothiopyran-3-yl | Propyl | $CH_2-CH=CH_2$ | 1-Imidazolyl |
| 122 | Tetrahydrothiopyran-3-yl | Propyl | $(E)-CH_2-CH=CH-CH_3$ | Cl |
| 123 | Tetrahydrothiopyran-3-yl | Propyl | $(E)-CH_2-CH=CH-CH_3$ | F |
| 124 | Tetrahydrothiopyran-3-yl | Propyl | $(E)-CH_2-CH=CH-CH_3$ | Br |

Tabelle 1 (Fortsetzung)

| Verbindung Nr. | $R^1$ | $R^2$ | $R^3$ | X |
|---|---|---|---|---|
| 125 | Tetrahydrothiopyran-3-yl | Propyl | (E)-$CH_2$-CH=CH-$CH_3$ | $NH_2$ |
| 126 | Tetrahydrothiopyran-3-yl | Propyl | (E)-$CH_2$-CH=CH-$CH_3$ | $NHCH_3$ |
| 127 | Tetrahydrothiopyran-3-yl | Propyl | (E)-$CH_2$-CH=CH-$CH_3$ | $N(CH_3)_2$ |
| 128 | Tetrahydrothiopyran-3-yl | Propyl | (E)-$CH_2$-CH=CH-$CH_3$ | $CH_3S$ |
| 129 | Tetrahydrothiopyran-3-yl | Propyl | (E)-$CH_2$-CH=CH-$CH_3$ | n-$C_3H_7S$ |
| 130 | Tetrahydrothiopyran-3-yl | Propyl | (E)-$CH_2$-CH=CH-$CH_3$ | Phenylthio |
| 131 | Tetrahydrothiopyran-3-yl | Propyl | (E)-$CH_2$-CH=CH-$CH_3$ | 1-Pyrazolyl |
| 132 | Tetrahydrothiopyran-3-yl | Propyl | (E)-$CH_2$-CH=CH-$CH_3$ | 1,2,4-Triazol-1-yl |
| 133 | Tetrahydrothiopyran-3-yl | Propyl | (E)-$CH_2$-CH=CH-$CH_3$ | 1-Pyrrolidinyl |
| 134 | Tetrahydrothiopyran-3-yl | Propyl | (E)-$CH_2$-CH=CH-$CH_3$ | N-Morpholino |
| 135 | Tetrahydrothiopyran-3-yl | Propyl | (E)-$CH_2$-CH=CH-$CH_3$ | Isopropylamino |
| 136 | Tetrahydrothiopyran-3-yl | Propyl | (E)-$CH_2$-CH=CH-$CH_3$ | Phenylamino |
| 137 | Tetrahydrothiopyran-3-yl | Propyl | (E)-$CH_2$-CH=CH-$CH_3$ | 1-Imidazolyl |
| 138 | Tetrahydrothiopyran-3-yl | Propyl | (E)-$CH_2$-CH=CH-Cl | Cl |
| 139 | Tetrahydrothiopyran-3-yl | Propyl | (E)-$CH_2$-CH=CH-Cl | F |
| 140 | Tetrahydrothiopyran-3-yl | Propyl | (E)-$CH_2$-CH=CH-Cl | Br |
| 141 | Tetrahydrothiopyran-3-yl | Propyl | (E)-$CH_2$-CH=CH-Cl | $NH_2$ |
| 142 | Tetrahydrothiopyran-3-yl | Propyl | (E)-$CH_2$-CH=CH-Cl | $NHCH_3$ |
| 143 | Tetrahydrothiopyran-3-yl | Propyl | (E)-$CH_2$-CH=CH-Cl | $N(CH_3)_2$ |
| 144 | Tetrahydrothiopyran-3-yl | Propyl | (E)-$CH_2$-CH=CH-Cl | $CH_3S$ |
| 145 | Tetrahydrothiopyran-3-yl | Propyl | (E)-$CH_2$-CH=CH-Cl | n-$C_3H_7S$ |

Tabelle 1 (Fortsetzung)

| Verbindung Nr. | $R^1$ | $R^2$ | $R^3$ | X |
|---|---|---|---|---|
| 146 | Tetrahydrothiopyran-3-yl | Propyl | (E)-$CH_2$-CH=CH-Cl | Phenylthio |
| 147 | Tetrahydrothiopyran-3-yl | Propyl | (E)-$CH_2$-CH=CH-Cl | 1-Pyrazolyl |
| 148 | Tetrahydrothiopyran-3-yl | Propyl | (E)-$CH_2$-CH=CH-Cl | 1,2,4-Triazol-1-yl |
| 149 | Tetrahydrothiopyran-3-yl | Propyl | (E)-$CH_2$-CH=CH-Cl | 1-Pyrrolidinyl |
| 150 | Tetrahydrothiopyran-3-yl | Propyl | (E)-$CH_2$-CH=CH-Cl | N-Morpholino |
| 151 | Tetrahydrothiopyran-3-yl | Propyl | (E)-$CH_2$-CH=CH-Cl | Isopropylamino |
| 152 | Tetrahydrothiopyran-3-yl | Propyl | (E)-$CH_2$-CH=CH-Cl | Phenylamino |
| 153 | Tetrahydrothiopyran-3-yl | Propyl | (E)-$CH_2$-CH=CH-Cl | 1-Imidazolyl |
| 154 | Tetrahydrothiopyran-3-yl | Propyl | $CH_2$-$CH_2$Cl | Cl |
| 155 | Tetrahydrothiopyran-3-yl | Propyl | $CH_2$-$CH_2$Cl | F |
| 156 | Tetrahydrothiopyran-3-yl | Propyl | $CH_2$-$CH_2$Cl | Br |
| 157 | Tetrahydrothiopyran-3-yl | Propyl | $CH_2$-$CH_2$Cl | $NH_2$ |
| 158 | Tetrahydrothiopyran-3-yl | Propyl | $CH_2$-$CH_2$Cl | $NHCH_3$ |
| 159 | Tetrahydrothiopyran-3-yl | Propyl | $CH_2$-$CH_2$Cl | $N(CH_3)_2$ |
| 160 | Tetrahydrothiopyran-3-yl | Propyl | $CH_2$-$CH_2$Cl | $CH_3S$ |
| 161 | Tetrahydrothiopyran-3-yl | Propyl | $CH_2$-$CH_2$Cl | n-$C_3H_7S$ |
| 162 | Tetrahydrothiopyran-3-yl | Propyl | $CH_2$-$CH_2$Cl | Phenylthio |
| 163 | Tetrahydrothiopyran-3-yl | Propyl | $CH_2$-$CH_2$Cl | 1-Pyrazolyl |
| 164 | Tetrahydrothiopyran-3-yl | Propyl | $CH_2$-$CH_2$Cl | 1,2,4-Triazol-1-yl |
| 165 | Tetrahydrothiopyran-3-yl | Propyl | $CH_2$-$CH_2$Cl | 1-Pyrrolidinyl |

Tabelle 1 (Fortsetzung)

| Verbindung Nr. | $R^1$ | $R^2$ | $R^3$ | X |
|---|---|---|---|---|
| 166 | Tetrahydrothiopyran-3-yl | Propyl | $CH_2-CH_2Cl$ | N-Morpholino |
| 167 | Tetrahydrothiopyran-3-yl | Propyl | $CH_2-CH_2Cl$ | Isopropylamino |
| 168 | Tetrahydrothiopyran-3-yl | Propyl | $CH_2-CH_2Cl$ | Phenylamino |
| 169 | Tetrahydrothiopyran-3-yl | Propyl | $CH_2-CH_2Cl$ | 1-Imidazoyl |
| 170 | Tetrahydrothiopyran-3-yl | Propyl | $CH_2-C\equiv CH$ | Cl |
| 171 | Tetrahydrothiopyran-3-yl | Propyl | $CH_2-C\equiv CH$ | F |
| 172 | Tetrahydrothiopyran-3-yl | Propyl | $CH_2-C\equiv CH$ | Br |
| 173 | Tetrahydrothiopyran-3-yl | Propyl | $CH_2-C\equiv CH$ | $NH_2$ |
| 174 | Tetrahydrothiopyran-3-yl | Propyl | $CH_2-C\equiv CH$ | $NHCH_3$ |
| 175 | Tetrahydrothiopyran-3-yl | Propyl | $CH_2-C\equiv CH$ | $N(CH_3)_2$ |
| 176 | Tetrahydrothiopyran-3-yl | Propyl | $CH_2-C\equiv CH$ | $CH_3S$ |
| 177 | Tetrahydrothiopyran-3-yl | Propyl | $CH_2-C\equiv CH$ | $n-C_3H_7S$ |
| 178 | Tetrahydrothiopyran-3-yl | Propyl | $CH_2-C\equiv CH$ | Phenylthio |
| 179 | Tetrahydrothiopyran-3-yl | Propyl | $CH_2-C\equiv CH$ | 1-Pyrazolyl |
| 180 | Tetrahydrothiopyran-3-yl | Propyl | $CH_2-C\equiv CH$ | 1,2,4-Triazol-1-yl |
| 181 | Tetrahydrothiopyran-3-yl | Propyl | $CH_2-C\equiv CH$ | 1-Pyrrolidinyl |
| 182 | Tetrahydrothiopyran-3-yl | Propyl | $CH_2-C\equiv CH$ | N-Morpholino |
| 183 | Tetrahydrothiopyran-3-yl | Propyl | $CH_2-C\equiv CH$ | Isopropylamino |
| 184 | Tetrahydrothiopyran-3-yl | Propyl | $CH_2-C\equiv CH$ | Phenylamino |
| 185 | Tetrahydrothiopyran-3-yl | Propyl | $CH_2-C\equiv CH$ | 1-Imidazolyl |
| 186 | 1-Oxo-tetrahydrothiopyran-3-yl | Ethyl | $C_2H_5$ | Cl |

Tabelle 1 (Fortsetzung)

| Verbindung Nr. | $R^1$ | $R^2$ | $R^3$ | X |
|---|---|---|---|---|
| 187 | 1-Oxo-tetrahydrothiopyran-3-yl | Propyl | $C_2H_5$ | Cl |
| 188 | 1,1-Dioxo-tetrahydrothiopyran-3-yl | Ethyl | $C_2H_5$ | Cl |
| 189 | 1,1-Dioxo-tetrahydrothiopyran-3-yl | Propyl | $C_2H_5$ | Cl |
| 190 | Tetrahydrothiopyran-4-yl | Ethyl | $C_2H_5$ | Cl |
| 191 | Tetrahydrothiopyran-4-yl | Propyl | $C_2H_5$ | Cl |
| 192 | $\Delta^3$-Dihydrothiopyran-3-yl | Ethyl | $C_2H_5$ | Cl |
| 193 | $\Delta^3$-Dihydrothiopyran-3-yl | Propyl | $C_2H_5$ | Cl |
| 194 | 5,5-Dimethyl-1,3-dioxan-2-yl | Ethyl | $C_2H_5$ | Cl |
| 195 | 5,5-Dimethyl-1,3-dioxan-2-yl | Propyl | $C_2H_5$ | Cl |
| 196 | 1,4-Dioxan-2-yl | Ethyl | $C_2H_5$ | Cl |
| 197 | 1,4-Dioxan-2-yl | Propyl | $C_2H_5$ | Cl |
| 198 | 1,4-Dithian-2-yl | Ethyl | $C_2H_5$ | Cl |
| 199 | 1,4-Dithian-2-yl | Propyl | $C_2H_5$ | Cl |
| 200 | 2-Pyridyl | Ethyl | $C_2H_5$ | Cl |
| 201 | 2-Pyridyl | Propyl | $C_2H_5$ | Cl |
| 202 | 3-Pyridyl | Ethyl | $C_2H_5$ | Cl |
| 203 | 3-Pyridyl | Propyl | $C_2H_5$ | Cl |
| 204 | 3-Pyridyl | Propyl | $C_2H_5$ | $NH_2$ |
| 205 | 3-Pyridyl | Propyl | $C_2H_5$ | $N(CH_3)_2$ |
| 206 | 3-Pyridyl | Propyl | $C_2H_5$ | 1-Pyrazolyl |
| 207 | 3-Pyridyl | Propyl | $C_2H_5$ | 1,2,4-Triazol-1-yl |

**Tabelle 1** (Fortsetzung)

| Verbindung Nr. | $R^1$ | $R^2$ | $R^3$ | X |
|---|---|---|---|---|
| 208 | 1,3-Dioxepan-5-yl | Ethyl | $C_2H_5$ | Cl |
| 209 | 1,3-Dioxepan-5-yl | Propyl | $C_2H_5$ | Cl |
| 210 | 2-Methyl-1,3-dioxepan-5-yl | Ethyl | $C_2H_5$ | Cl |
| 211 | 2-Methyl-1,3-dioxepan-5-yl | Propyl | $C_2H_5$ | Cl |
| 212 | 2-Isopropyl-1,3-dioxepan-5-yl | Ethyl | $C_2H_5$ | Cl |
| 213 | 2-Isopropyl-1,3-dioxepan-5-yl | Ethyl | $C_2H_5$ | $NH_2$ |
| 214 | 2-Isopropyl-1,3-dioxepan-5-yl | Ethyl | $C_2H_5$ | $NHCH_3$ |
| 215 | 2-Isopropyl-1,3-dioxepan-5-yl | Ethyl | $C_2H_5$ | $N(CH_3)_2$ |
| 216 | 2-Isopropyl-1,3-dioxepan-5-yl | Ethyl | $C_2H_5$ | 1-Pyrrolidino |
| 217 | 2-Isopropyl-1,3-dioxepan-5-yl | Ethyl | $C_2H_5$ | $C_2H_5S$ |
| 218 | 2-Isopropyl-1,3-dioxepan-5-yl | Ethyl | $C_2H_5$ | 1-Pyrazolyl |
| 219 | 2-Isopropyl-1,3-dioxepan-5-yl | Ethyl | $C_2H_5$ | 1,2,4-Triazol-1-yl |
| 220 | 2-Ethylthioprop-1-yl | Ethyl | $C_2H_5$ | Cl |
| 221 | 2-Ethylthioprop-1-yl | Propyl | $C_2H_5$ | Cl |
| 222 | 2-Ethylthioprop-1-yl | Propyl | $C_2H_5$ | $NH_2$ |
| 223 | 2-Ethylthioprop-1-yl | Propyl | $C_2H_5$ | $N(CH_3)_2$ |
| 224 | 2-Ethylthioprop-1-yl | Propyl | $C_2H_5$ | N-Morpholino |
| 225 | Ethoxycarbonyl | Propyl | $C_2H_5$ | Cl |
| 226 | Ethoxycarbonyl | Propyl | $C_2H_5$ | $NH_2$ |
| 227 | Ethoxycarbonyl | Propyl | $C_2H_5$ | 1-Pyrazolyl |
| 228 | Ethoxycarbonyl | Propyl | $C_2H_5$ | 1,2,4-Triazolo-1-yl |
| 370 | Tetrahydropyran-4-yl | Propyl | (E)-$CH_2$-CH=CH-Cl | |
| 371 | Tetrahydropyran-4-yl | Propyl | (E)-$CH_2$-CH=CH-Cl | N-Morpholino |
| 372 | 6-Methoxy-tetrahydropyran-3-yl | Propyl | $C_2H_5$ | Cl |
| 373 | 3-Isopropyl-isoxazol-5-yl | Ethyl | $C_2H_5$ | $NHCH_3$ |

Tabelle 2

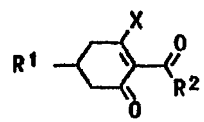

| Verbindung Nr. | $R^1$ | $R^2$ | X |
|---|---|---|---|
| 229 | Tetrahydrofuran-2-yl | Ethyl | Cl |
| 230 | Tetrahydrofuran-2-yl | Propyl | Cl |
| 231 | Tetrahydrofuran-3-yl | Ethyl | Cl |
| 232 | Tetrahydrofuran-3-yl | Propyl | Cl |
| 233 | Tetrahydrofuran-3-yl | Propyl | $NH_2$ |
| 234 | Tetrahydrofuran-3-yl | Propyl | $NHCH_3$ |
| 235 | Tetrahydrofuran-3-yl | Propyl | 1-Pyrazolyl |
| 236 | Tetrahydrofuran-3-yl | Propyl | $C_2H_5S$ |
| 237 | Tetrahydrofuran-3-yl | Ethyl | Cl |
| 238 | Tetrahydrofuran-3-yl | Propyl | Cl |
| 239 | 2,2-Dimethyl-2,5-dihydrothien-3-yl | Ethyl | Cl |
| 240 | 2,2-Dimethyl-2,5-dihydrothien-3-yl | Propyl | Cl |
| 241 | 2-Furyl | Ethyl | Cl |
| 242 | 2-Furyl | Propyl | Cl |
| 243 | 2-Furyl | Propyl | $NH_2$ |
| 244 | 2-Furyl | Propyl | 1-Pyrrolidinyl |
| 245 | 2-Furyl | Propyl | N-Morpholino |

Tabelle 2 (Fortsetzung)

| Verbindung Nr. | $R^1$ | $R^2$ | X |
|---|---|---|---|
| 246 | 2-Furyl | Propyl | Phenylamino |
| 247 | 3-Furyl | Ethyl | Cl |
| 248 | 3-Furyl | Propyl | Cl |
| 249 | 2-Thienyl | Ethyl | Cl |
| 250 | 2-Thienyl | Propyl | Cl |
| 251 | 3-Thienyl | Ethyl | Cl |
| 252 | 3-Thienyl | Propyl | Cl |
| 253 | 2,5-Dimethyl-thien-3-yl | Ethyl | Cl |
| 254 | 2,5-Dimethyl-thien-3-yl | Propyl | Cl |
| 255 | 3-Methyl-isoxazol-5-yl | Ethyl | Cl |
| 256 | 3-Methyl-isoxazol-5-yl | Propyl | Cl |
| 257 | 3-Isopropyl-isoxazol-5-yl | Ethyl | Cl |
| 258 | 3-Isopropyl-isoxazol-5-yl | Propyl | Cl |
| 259 | 4-Methyl-isothiazol-5-yl | Ethyl | Cl |
| 260 | 4-Methyl-isothiazol-5-yl | Propyl | Cl |
| 261 | Isothiazol-4-yl | Ethyl | Cl |
| 262 | Isothiazol-4-yl | Propyl | Cl |
| 263 | 2-Methyl-thiazol-4-yl | Ethyl | Cl |
| 264 | 2-Methyl-thiazol-4-yl | Propyl | Cl |
| 265 | 2-Methyl-thiazol-4-yl | Ethyl | Cl |
| 266 | 2-Methyl-thiazol-4-yl | Propyl | Cl |
| 267 | 2-Phenyl-thiazol-4-yl | Ethyl | Cl |

Tabelle 2 (Fortsetzung)

| Verbindung Nr. | $R^1$ | $R^2$ | X |
|---|---|---|---|
| 268 | Tetrahydropyran-2-yl | Ethyl | Cl |
| 269 | Tetrahydropyran-2-yl | Propyl | Cl |
| 270 | 6-Methyl-tetrahydropyran-2-yl | Ethyl | Cl |
| 271 | 6-Methyl-tetrahydropyran-2-yl | Propyl | Cl |
| 272 | 6-Methoxy-tetrahydropyran-2-yl | Ethyl | Cl |
| 273 | 6-Methoxy-tetrahydropyran-2-yl | Propyl | Cl |
| 274 | 6-Ethoxy-tetrahydropyran-2-yl | Ethyl | Cl |
| 275 | 6-Ethoxy-tetrahydropyran-2-yl | Propyl | Cl |
| 276 | Tetrahydropyran-3-yl | Methyl | Cl |
| 277 | Tetrahydropyran-3-yl | Ethyl | Cl |
| 278 | Tetrahydropyran-3-yl | Propyl | Cl |
| 279 | Tetrahydropyran-3-yl | Propyl | $NH_2$ |
| 280 | Tetrahydropyran-3-yl | Propyl | $NHCH_3$ |
| 281 | Tetrahydropyran-3-yl | Propyl | $C_2H_5S$ |
| 282 | Tetrahydropyran-3-yl | Propyl | 1,2,4-Triazol-1-yl |
| 283 | Tetrahydropyran-3-yl | Propyl | 1-Pyrazolyl |
| 284 | Tetrahydropyran-3-yl | Propyl | 1-Imidazolyl |
| 285 | Tetrahydropyran-3-yl | Propyl | N-Piperidyl |
| 286 | Tetrahydropyran-3-yl | Propyl | N-Morpholino |
| 287 | 4-Methyl-tetrahydropyran-3-yl | Ethyl | Cl |
| 288 | 4-Methyl-tetrahydropyran-3-yl | Propyl | Cl |
| 289 | 2-Methoxy-tetrahydropyran-3-yl | Ethyl | Cl |
| 290 | 2-Methoxy-tetrahydropyran-3-yl | Propyl | Cl |

**Tabelle 2** (Fortsetzung)

| Verbindung Nr. | $R^1$ | $R^2$ | X |
|---|---|---|---|
| 291 | Tetrahydropyran-4-yl | Methyl | Cl |
| 292 | Tetrahydropyran-4-yl | Ethyl | Cl |
| 293 | Tetrahydropyran-4-yl | Propyl | Cl |
| 294 | Tetrahydropyran-4-yl | Isopropyl | Cl |
| 295 | Tetrahydropyran-4-yl | Butyl | Cl |
| 296 | Tetrahydropyran-4-yl | Isobutyl | Cl |
| 297 | Tetrahydropyran-4-yl | Ethyl | $NH_2$ |
| 298 | Tetrahydropyran-4-yl | Ethyl | $NHCH_3$ |
| 299 | Tetrahydropyran-4-yl | Ethyl | $N(CH_3)_2$ |
| 300 | Tetrahydropyran-4-yl | Ethyl | Phenylamino |
| 301 | Tetrahydropyran-4-yl | Ethyl | Isopropylamino |
| 302 | Tetrahydropyran-4-yl | Ethyl | 1-Pyrrolidinyl |
| 303 | Tetrahydropyran-4-yl | Ethyl | $C_2H_5S$ |
| 304 | Tetrahydropyran-4-yl | Ethyl | 1-Pyrazol |
| 305 | Tetrahydropyran-4-yl | Ethyl | 1,2,4-Triazol-1-yl |
| 306 | 3-Methyl-tetrahydropyran-4-yl | Ethyl | Cl |
| 307 | 3-Methyl-tetrahydropyran-4-yl | Propyl | Cl |
| 308 | $\Delta^3$-Dihydropyran-3-yl | Ethyl | Cl |
| 309 | $\Delta^3$-Dihydropyran-3-yl | Propyl | Cl |
| 310 | Tetrahydrothiopyran-3-yl | Methyl | Cl |
| 311 | Tetrahydrothiopyran-3-yl | Ethyl | Cl |
| 312 | Tetrahydrothiopyran-3-yl | Propyl | Cl |

Tabelle 2 (Fortsetzung)

| Verbindung Nr. | $R^1$ | $R^2$ | X |
|---|---|---|---|
| 313 | Tetrahydrothiopyran-3-yl | Isopropyl | Cl |
| 314 | Tetrahydrothiopyran-3-yl | Butyl | Cl |
| 315 | Tetrahydrothiopyran-3-yl | Isobutyl | Cl |
| 316 | Tetrahydrothiopyran-3-yl | Propyl | F |
| 317 | Tetrahydrothiopyran-3-yl | Propyl | Br |
| 318 | Tetrahydrothiopyran-3-yl | Propyl | $NH_2$ |
| 319 | Tetrahydrothiopyran-3-yl | Propyl | $NHCH_3$ |
| 320 | Tetrahydrothiopyran-3-yl | Propyl | $N(CH_3)_2$ |
| 321 | Tetrahydrothiopyran-3-yl | Propyl | $CH_3S$ |
| 322 | Tetrahydrothiopyran-3-yl | Propyl | $C_2H_5S$ |
| 323 | Tetrahydrothiopyran-3-yl | Propyl | n-$C_3H_7S$ |
| 324 | Tetrahydrothiopyran-3-yl | Propyl | Phenylthio |
| 325 | Tetrahydrothiopyran-3-yl | Propyl | 1-Pyrazolyl |
| 326 | Tetrahydrothiopyran-3-yl | Propyl | 1,2,4-Triazol-1-yl |
| 327 | 1-Oxo-tetrahydrothiopyran-3-yl | Ethyl | Cl |
| 328 | 1-Oxo-tetrahydrothiopyran-3-yl | Propyl | Cl |
| 329 | 1,1-Dioxo-tetrahydrothiopyran-3-yl | Ethyl | Cl |
| 330 | 1,1-Dioxo-tetrahydrothiopyran-3-yl | Propyl | Cl |
| 331 | Tetrahydrothiopyran-4-yl | Ethyl | Cl |
| 332 | Tetrahydrothiopyran-4-yl | Propyl | Cl |
| 333 | $\Delta^3$-Dihydrothiopyran-3-yl | Ethyl | Cl |
| 334 | $\Delta^3$-Dihydrothiopyran-3-yl | Propyl | Cl |
| 335 | 5,5-Dimethyl-1,3-dioxan-2-yl | Ethyl | Cl |

Tabelle 2 (Fortsetzung)

| Verbindung Nr. | $R^1$ | $R^2$ | X |
|---|---|---|---|
| 336 | 5,5-Dimethyl-1,3-dioxan-2-yl | Propyl | Cl |
| 337 | 1,4-Dioxan-2-yl | Ethyl | Cl |
| 338 | 1,4-Dioxan-2-yl | Propyl | Cl |
| 339 | 1,4-Dithian-2-yl | Ethyl | Cl |
| 340 | 1,4-Dithian-2-yl | Propyl | Cl |
| 341 | 2-Pyridyl | Ethyl | Cl |
| 342 | 2-Pyridyl | Propyl | Cl |
| 343 | 3-Pyridyl | Ethyl | Cl |
| 344 | 3-Pyridyl | Propyl | Cl |
| 345 | 3-Pyridyl | Propyl | $NH_2$ |
| 346 | 3-Pyridyl | Propyl | $N(CH_3)_2$ |
| 347 | 3-Pyridyl | Propyl | 1-Pyrazolyl |
| 348 | 3-Pyridyl | Propyl | 1,2,4-Triazol-1-yl |
| 349 | 1,3-Dioxepan-5-yl | Ethyl | Cl |
| 350 | 1,3-Dioxepan-5-yl | Propyl | Cl |
| 351 | 2-Methyl-1,3-dioxepan-5-yl | Ethyl | Cl |
| 352 | 2-Methyl-1,3-dioxepan-5-yl | Propyl | Cl |
| 353 | 2-Isopropyl-1,3-dioxepan-5-yl | Ethyl | Cl |
| 354 | 2-Isopropyl-1,3-dioxepan-5-yl | Ethyl | $NH_2$ |
| 355 | 2-Isopropyl-1,3-dioxepan-5-yl | Ethyl | $NHCH_3$ |
| 356 | 2-Isopropyl-1,3-dioxepan-5-yl | Ethyl | $N(CH_3)_2$ |
| 357 | 2-Isopropyl-1,3-dioxepan-5-yl | Ethyl | 1-Pyrrolidino |

<u>Tabelle 2</u> (Fortsetzung)

| Verbindung Nr. | $R^1$ | $R^2$ | X |
|---|---|---|---|
| 358 | 2-Isopropyl-1,3-dioxepan-5-yl | Ethyl | $C_2H_5S$ |
| 359 | 2-Isopropyl-1,3-dioxepan-5-yl | Ethyl | 1-Pyrazolyl |
| 360 | 2-Isopropyl-1,3-dioxepan-5-yl | Ethyl | 1,2,4-Triazol-1-yl |
| 361 | 2-Ethylthioprop-1-yl | Ethyl | Cl |
| 362 | 2-Ethylthioprop-1-yl | Propyl | Cl |
| 363 | 2-Ethylthioprop-1-yl | Propyl | $NH_2$ |
| 364 | 2-Ethylthioprop-1-yl | Propyl | $N(CH_3)_2$ |
| 365 | 2-Ethylthioprop-1-yl | Propyl | N-Morpholino |
| 366 | Ethoxycarbonyl | Propyl | Cl |
| 367 | Ethoxycarbonyl | Propyl | $NH_2$ |
| 368 | Ethoxycarbonyl | Propyl | 1-Pyrazolyl |
| 369 | Ethoxycarbonyl | Propyl | 1,2,4-Triazol-1-yl |
| 374 | Tetrahydrothiopyran-3-yl | Propyl | Pyrrolidinyl |
| 375 | 1-Oxo-tetrahydrothiopyran-3-yl | Propyl | Pyrrolidinyl |
| 376 | 1,1-Dioxo-tetrahydrothiopyran-3-yl | Propyl | Pyrrolidinyl |
| 377 | Ethoxycarbonyl | Propyl | $N(CH_3)_2$ |
| 378 | Ethoxycarbonyl | Propyl | $NH_2$ |
| 379 | Ethoxycarbonyl | Propyl | $n-C_3H_7-S$ |
| 380 | Ethoxycarbonyl | Propyl | $NH-CH(CH_3)_2$ |

Die Identifizierung und Charakterisierung der Verbindungen der Formel I erfolgen am geeignetsten mit Hilfe der Protonenresonanzspektroskopie. In der nachstehenden Tabelle sind daher einige strukturspezifische $^1$H-NMR-Daten angegeben (Lösungsmittel $CDCl_3$, interner Standard Tetramethylsilan; s = Singulett, d = Dublett, t = triplett, q = Quartett, m = Multiplett).

| Wirkstoff-Nr. | Charakteristische $^1$H-NMR-Daten in ppm |
|---|---|
| 22 | 0,97 (t); 4,17 (q); 6,87 (d); 6,98 (dd); 7,22 (d) |
| 29 | 0,92 (t); 1,27 (m); 4,17 (q); 5,95 (s) |
| 53 | 0,92 (t); 1,26 (t); 3,88 (t); 4,14 (q) |
| 71 | 0,96 (t); 3,38 (t); 4,01 (d); 4,59 (d) |
| 73 | 1,13 (t); 2,93 (s); 3,99 (m); 4,50 (d); 6,28 (d) |
| 74 | 1,07 (t); 2,98 (s); 3,40 (t); 4,59 (d); 6,30 (d) |
| 76 | 1,07 (t); 1,24 (d); 3,39 (t); 4,55 (d); 6,26 (d) |
| 77 | 1,03 (t); 3,33 (m); 4,01 (d); 4,56 (d); 6,26 (d) |
| 90 | 0,97 (t); 1,30 (t); 2,0–3,0 (m) (t); 4,20 (q) |
| 93 | 0,89 (t); 1,23 (t); 4,12 (q) |
| 94 | 0,88 (t); 1,25 (t); 2,95 (d); 4,07 (q) |
| 95 | 0,92 (t); 1,26 (t); 2,95 (s); 4,13 (q) |
| 96 | 0,92 (t); 2,42 (s); 4,05 (q, anti); 4,15 (q, syn) |
| 101 | 0,93 (t); 3,42 (m); 4,13 (q) |
| 102 | 0,92 (t); 3,33 (m); 3,70 (t); 4,14 (q) |
| 103 | 0,95 (t); 3,78 (m); 4,08 (q); 8,32 (NH) |
| 122 | 0,92 (t); 1,71 (d); 4,5 (m); 5,67 (m) |
| 170 | 0,93 (t); 1,40 (m); 4,67 (s) |
| 221 | 0,92 (m); 2,82 (m); 4,14 (q); 4,14 (q) |
| 225 | 0,92 (t); 2,42 (m); 4,17 (2q) |
| 250 | 1,13 (t); 3,78 (m); 6,88 (s); 6,97 (dd); 7,22 (d) |
| 292 | 1,13 (t); 3,77 (t); 4,02 (d) |
| 297 | 1,04 (t); 2,97 (m); 3,37 (t); 4,01 (d) |
| 298 | 1,07 (t); 3,05 (d); 3,37 (t); 4,02 (d) |
| 299 | 1,03 (t); 3,02 (s); 3,37 (t); 4,00 (d) |
| 301 | 1,07 (t); 1,32 (t); 3,38 (t); 3,87 (m); 4,02 (d) |
| 302 | 1,07 (t); 3,37 (t); 3,98 (d) |
| 304 | 1,17 (t); 3,42 (t); 4,04 (d); 6,45 (s); 7,67 (s); 7,73 (dd) |
| 305 | 1,14 (t); 3,43 (t); 8,04 (s); 8,47 (s) |
| 310 | 1,27 (m); 1,62 (m); 2,37 (s); 3,89 (t) |
| 312 | 0,98 (t); 1,23 (m); 1,5–2,9 (m) |
| 318 | 0,97 (t); 2,93 (m); 6,18 (s); 11,19 (s) |
| 319 | 0,95 (t); 2,95 (t); 3,10 ($NCH_3$); 12,5 (s) |
| 320 | 0,95 (t); 1,55–2,95 (m); 3,04 (s) |
| 323 | 0,98 (t); 1,06 (t); 2,0–3,0 (m) |
| 324 | 1,01 (t); 2,81 (t); 7,45 (m) |
| 325 | 0,96 (t); 3,08 (d); 6,47 (s); 7,68 (s); 7,78 (s) |
| 326 | 0,96 (t); 3,07 (d); 8,06 (s); 8,48 (s) |
| 367 | 0,92 (t); 1,25 (t); 1,60 (m); 4,19 (q) |
| 368 | 0,92 (t); 1,28 (t); 4,22 (q); 6,46 (m); 6,68 (d); 7,77 (d) |
| 369 | 0,95 (t); 1,26 (t); 2,58 (t); 8,05 (s); 8,51 (s) |
| 330 | 0,95 (t); 1,68 (q); 2,58 (t) |
| 370 | 0,90 (t); 3,38 (t); 4,57 (d) |
| 371 | 0,95 (t); 4,59 (d); 6,31 (d) |
| 372 | 0,92 (t); 1,26 (t); 3,37 (s); 4,14 (q) |
| 373 | 1,01 (t); 4,09 (q); 5,96 (s) |
| 374 | 0,95 (t); 2,2–2,8 (m); 2,9–3,05 (m) |
| 375 | 0,96 (t); 1,1–3,8 (m) |
| 377 | 0,94 (t); 1,27 (t); 3,03 (s) |
| 378 | 0,92 (t); 1,25 (t); 1,60 (m); 4,19 (q) |
| 379 | 0,97 (t); 1,08 (t); 1,31 (t); 4,23 (q) |

| Wirkstoff-Nr. | Charakteristische $^1$H-NMR-Daten in ppm |
|---|---|
| 380 | 0,95 (t); 1,36 (d); 4,18 (q) |

Die Cyclohexanonderivate der Formel I können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel, wie N,N-Dimethylformamid, Dimethylsulfoxid, N-Methylpyrrolidon oder Wasser in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali-und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol, Octylphenol oder Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetat, Sorbitester, Lignin, Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs-und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten zwischen 0,1 und 95 Gew.%, vorzugsweise zwischen 0,5 und 90 Gew.%, Wirkstoff.

Beispiele für Formulierungen sind:

I. Man vermischt 90 Gewichtsteile der Verbindung Nr. 71 mit 10 Gewichtsteilen N-Methyl-α-pyrrolidon und erhält eine Lösung, die zur Anwendung in Form kleinster Tropfen geeignet ist.

II. 20 Gewichtsteile der Verbindung Nr. 102 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.

III. 20 Gewichtsteile der Verbindung Nr. 90 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

IV. 20 Gewichtsteile des Wirkstoffs Nr. 53 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Rizinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

V. 20 Gewichtsteile des Wirkstoffs Nr. 310 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffs enthält.

VI. 3 Gewichtsteile des Wirkstoffs Nr. 366 werden mit 97 Gewichtsteilen feinteiligem Kaolin vermischt. Man erhält auf diese Weise ein Stäubemittel, das 3 Gew.% des Wirkstoffs enthält.

VII. 30 Gewichtsteile des Wirkstoffs Nr. 325 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Gewichtsteile des Wirkstoffs Nr. 310 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.

IX. 40 Gew.-Teile des Wirkstoffs Nr. 53 werden in 60 Gewichtsteilen einer Mischung, die aus 93 Gew.% Xylol und 7 Gew.% des Anlagerungsproduktes von 8 Mol Ethylenoxid an 1 Mol Nonylphenol besteht, gelöst. Man erhält eine Lösung, die 40 Gew.% des Wirkstoffs enthält.

Die neuen Cyclohexenonderivate der Formel I, in der A für den Rest $NOR^3$ steht, besitzen eine gute herbizide Wirkung vorzugsweise gegen Arten aus der Familie der Gräser (Gramineen). Sie sind verträglich und somit selektiv in breitblättrigen Kulturen sowie in monokotylen Gewächsen, welche nicht zu den Gramineen zählen. Ferner sind Verbindungen darunter, welche sich auch n Gramineenkulturen wie Weizen und Reis selektiv verhalten und gleichzeitig unerwünschte Gräser bekämpfen.

Die Applikation der Mittel kann im Vorauflaufverfahren oder im Nachauflaufverfahren erfolgen. Sind die Wirkstoffe für gewisse Kulturpflanzen weniger verträglich, so können auch Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (postdirected, lay-by).

Die Aufwandmengen an Wirkstoff betragen je nach Jahreszeit, Zielpflanzen und Wachstumsstadium 0,025 bis 3kg/ha, vorzugsweise 0,05 bis 1,0 kg/ha.

Die Wirkung der Cyclohexenonderivate der Formel I ($A = NOR^3$) auf das Pflanzenwachstum läßt sich durch Gewächshausversuche zeigen:

Als Kulturgefäße dienen Plastikblumentöpfe mit 300 $cm^3$ Inhalt und lehmigem Sand mit etwa 3,0% Humus als Substrat. Die Samen der Testpflanzen werden nach Arten getrennt flach eingesät. Bei Vorauflaufbehandlung werden die Wirkstoffe unmittelbar danach auf die Erdoberfläche aufgebracht. Sie werden hierbei in Wasser als Verteilungsmittel suspendiert oder emulgiert und mittels fein verteilender Düsen gespritzt. Die Aufwandmengen betragen 3,0 kg/ha.

Nach dem Aufbringen der Mittel werden die Gefäße leicht beregnet, um Keimung und Wachstum in Gang zu bringen. Danach deckt man die Gefäße mit durchsichtigen Plastikhauben ab, bis die Pflanzen angewachsen sind. Diese Abdeckung bewirkt ein gleichmäßiges Keimen der Testpflanzen, sofern dies nicht durch die Wirkstoffe beeinträchtigt wird.

Zum Zwecke der Nachauflaufbehandlung zieht man die Testpflanzen je nach Wuchsform erst bis zu einer Wuchshöhe von 3 bis 15 cm an und behandelt sie danach. Die Sojapflanzen werden in einem mit Torfmull (peat) angereicherten Substrat angezogen. Zur Nachauflaufbehandlung werden entweder direkt gesäte und in den gleichen Gefäßen aufgewachsene Pflanzen ausgewählt, oder sie werden erst als Keimpflanzen getrennt angezogen und einige Tage vor der Behandlung in die Versuchsgefäße verpflanzt. Die Aufwandmenge für die Nachauflaufbehandlung beträgt 0,5 bis 1,0 kg Wirkstoff/ha. Eine Abdeckung unterbleibt bei der Nachauflaufbehandlung.

Die Versuchsgefäße werden im Gewächshaus aufgestellt, wobei für wärmeliebende Arten wärmere Bereiche (20 bis 35°C) und für solche gemäßigter Klimata 10 bis 25°C bevorzugt werden. Die Versuchsperiode erstreckt sich über 2 bis 4 Wochen. Während dieser Zeit werden die Pflanzen gepflegt und ihre Reaktion auf die einzelnen Behandlungen wird ausgewertet. Bewertet wird nach einer Skala von 0 bis 100. Dabei bedeutet 100 kein Aufgang der Pflanzen bzw. völlige Zerstörung zumindest der oberirdischen Teile.

Die in den Gewächshausversuchen verwendeten Pflanzen setzen sich aus folgenden Arten zusammen:

| Lat. Name | Deutscher Name |
|---|---|
| Alopecurus myosuroides | Ackerfuchsschwanz |
| Avena sativa | Hafer |
| Glycine max | Sojabohnen |
| Lolium multiflorum | Ital. Raygras |
| Setaria italica | Kolbenhirse |
| Sinapis alba | Weißer Senf |
| Zea mays | Mais |
| Avena fatua | Flughafer |
| Digitaria sanguinalis | Blutfingerhirse |
| Echinochloa crus-galli | Hühnerhirse |
| Medicago sativa | Luzerne |
| Triticum aestivum | Weizen |

Mit den beispielhaft ausgewählten Wirkstoffen Nr. 90, 102 und 71 lassen sich bei Vorauflaufapplikation von 3 kg Wirkstoff/ha Pflanzen aus der Familie der Gramineen gut bekämpfen. Senf als breitblättrige Beispielpflanze erleidet keinerlei Schädigung.

Im Nachauflaufverfahren sind beispielsweise die Verbindungen Nr. 53 und 90 gut zur Bekämpfung unerwünschten Graswuchses geeignet; auch Ausfallkulturgräser wie z.B. Mais werden erfaßt. Die breitblättrige Kulturpflanze Soja bleibt dabei völlig unbeeinflußt.

Im Nachauflaufverfahren sind beispielsweise die Verbindungen 71 und 102 zur Bekämpfung von Gräsern ohne Schädigung der Kulturpflanze Luzerne bei einer Aufwandmenge von 1 kg Wirkstoff je ha geeignet.

Bei einer Aufwandmenge von 0,5 kg Wirkstoff je ha im Nachauflauf werden beispielsweise von den Verbindungen 212 und 371 unerwünschte Hirsen vernichtet, während Weizen fast nicht geschädigt wird.

Der Wirkstoff 122 schädigt beispielsweise bei einer Aufwandmenge von 1kg/ha unerwünschte Gräser stark, während die Nutzpflanze Luzerne nicht geschädigt wird.

Bei Aufwandmengen von 3 kg Wirkstoff/ha schädigen beispielsweise die Verbindungen 71, 96, 102, 122 und 371 Grasarten stark, während die breitblättrige Pflanze Senf nicht geschädigt wird.

In Anbetracht des erfaßbaren Wirkungsspektrums zur Unkrautbekämpfung der Verträglichkeit für Kulturpflanzen oder der erwünschten Beeinflussung des Wachstums derselben sowie angesichts der Vielfalt der Applikationsmethoden können die erfindungsgemäßen Verbindungen der Formel I (mit A = $NOR^3$) in einer großen Zahl von Kulturpflanzen eingesetzt werden.

Zur Verbreiterung des Wirkungsspektrums und zur Erzielung synergistischer Effekte können die Cyclohexenonderivate I sowohl unter sich als auch mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Chinolincarbonsäuren und andere in Betracht.

Außerdem kann es von Nutzen sein, die Cyclohexenonderivate der Formel I (A = $NOR^3$) allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

Die Cyclohexenonderivate der Formel I, in der A für Sauerstoff steht, können praktisch alle Entwicklungsstadien einer Pflanze verschiedenartig beeinflussen und werden deshalb als Wachstumsregulatoren eingesetzt. Die Wirkungsvielfalt der Pflanzenwachstumsregulatoren hängt ab vor allem

a) von der Pflanzenart und -sorte,
b) von dem Zeitpunkt der Applikation, bezogen auf das Entwicklungsstadium der Pflanze und von der Jahreszeit,
c) von dem Applikationsort und -verfahren (Samenbeize, Bodenbehandlung oder Blattapplikation),
d) von klimatischen Faktoren, z.B. Tageslänge, Lichtintensität, Durchschnittstemperatur, Niederschlagsmenge,
e) von der Bodenbeschaffenheit (einschließlich Düngung),
f) von der Formulierung bzw. Anwendungsform des Wirkstoffs und schließlich
g) von den angewendeten Konzentrationen der aktiven Substanz.

Aus der Reihe der verschiedenartigen Anwendungsmöglichkeiten der erfindungsgemäßen Pflanzenwachstumsregulatoren im Pflanzenanbau, in der Landwirtschaft und im Gartenbau, werden einige nachstehend erwähnt.

A. Mit den neuen Verbindungen läßt sich das vegetative Wachstum der Pflanzen stark hemmen, was sich insbesondere in einer Reduzierung des Längenwachstums äußert. Die behandelten Pflanzen weisen demgemäß einen gedrungenen Wachstum auf; außerdem ist eine dunklere Blattfärbung zu beobachten.
Als vorteilhaft für die Praxis erweist sich z.B. die Verringerung des Grasbnewuchses an Straßenrändern, Kanalböschungen und auf Rasenflächen wie Park-, Sport- und Obstanlagen, Zierrasen und Flugplätzen, so daß der arbeits- und kostenaufwendige Rasenschnitt reduziert werden kann.
Von wirtschaftlichem Interesse ist auch die Erhöhung der Standfestigkeit von lageranfälligen Kulturen wie Getreide, Mais, Sonnenblumen und Soja. Die dabei verursachte Halmverkürzung und Halmverstärkung verringern oder beseitigen die Gefahr des "Lagerns" (des Umknickens) von Pflanzen unter ungünstigen Witterungsbedingungen vor der Ernte.
Wichtig ist auch die Anwendung von Wachstumsregulatoren zur Hemmung des Längenwachstums und zur zeitlichen Veränderung des Reifeverlaufs bei Baumwolle. Damit wird ein vollstädig mechanisiertes Beernten dieser wichtigen Kulturpflanzen ermöglicht.
Durch Anwendung von Wachstumsregulatoren kann auch die seitliche Verzweigung der Pflanzen vermehrt oder gehemmt werden. Daran besteht Interesse, wenn z.B. bei Tabakpflanzen die Ausbildung von Seitentrieben (Geiztrieben) zugunsten des Blattwachstums gehemmt werden soll.
Mit Wachstumsregulatoren läßt sich beispielsweise bei Winterraps auch die Frostresistenz erheblich erhöhen. Dabei werden einerseits das Längenwachstum und die Entwicklung einer zu üppigen (und dadurch besonders frostanfälligen) Blatt- bzw. Pflanzenmasse gehemmt. Andererseits werden die jungen Rapspflanzen nach der Aussaat und vor dem Einsetzen der Winterfröste trotz günstiger Wachstumsbedingungen im vegetativen Entwicklungsstadium zurückgehalten. Dadurch wird auch die Frostgefährdung solcher Pflanzen beseitigt, die zum vorzeitigen Abbau der Blühhemmung und zum Übergang in die generative Phase neigen. Auch bei anderen Kulturen, z.B. Wintergetreide ist es vorteilhaft, wenn die Bestände durch Behandlung mit erfindungsgemäßen Verbindungen im Herbst zwar gut bestockt werden, aber nicht zu üppig in den Winter hineingehen. Dadurch kann der erhöhten Frostempfindlichkeit und - wegen der relativ geringen Blatt- bzw. Pflanzenmasse - dem Befall mit verschiedenen Krankheiten (z.B. Pilzkrankheit) vorgebeugt werden. Die Hemmung des vegetativen Wachstums ermöglicht außerdem bei vielen Kulturpflanzen eine dichtere Bepflanzung des Bodens, so daß ein Mehrertrag bezogen auf die Bodenfläche erzielt werden kann.

B. Mit den neuen Mitteln lassen sich Mehrerträge sowohl an Pflanzenteilen als auch an Pflanzeninhaltsstoffen erzielen. So ist es beispielsweise möglich, das Wachstum größerer Meengen an Knospen, Blüten, Blättern, Früchten, Samenkörnern, Wurzeln und Knollen zu induzieren, den Gehalt an Zucker in Zuckerrübeen, Zuckerrohr sowie Zitrusfrüchten zu erhöhten, den Proteingehalt in Getreide oder Soja zu steigern oder Gummibäume zum vermehrten Latexfluß zu stimulieren.
Dabei können die Cyclohexenonderivate der Formel I, in der A für Sauerstoff steht, Ertragssteigerungen durch Eingriffe in den pflanzlichen Stoffwechsel bzw. durch Förderung oder Hemmung des vegetativen und/oder des generativen Wachstums verursachen.

C. Mit Pflanzenwachstumsregulatoren lassen sich schließlich sowohl eine Verkürzung bzw. Verlängerung der Entwicklungsstadien als auch eine Beschleunigung bzw. Verzögerung der Reife der geernteten Pflanzenteile vor oder nach der Ernte erreichen.
Von wirtschaftlichem Interesse ist beispielsweise die Ernteerleichterung, die durch das zeitlich konzentrierte Abfallen oder Vermindern der Haftfestigkeit am Baum bei Zitrusfrüchten, Oliven oder bei anderen Arten und Sorten von Kern-, Stein- und Schalenobst ermöglicht wird. Derselbe Mechanismus, das heißt die Förderung der Ausbildung von Trenngewebe zwischen dem Blatt und dem Sproß der Pflanze ist auch für ein gut kontrollierbares Entblättern anderer Nutzpflanzen wesentlich.

D. Mit Wachstumsregulatoren kann weiterhin der Wasserverbrauch von Pflanzen reduziert werden. Dies ist besonders wichtig für landwirtschaftliche Nutzflächen, die unter einem hohen Kostenaufwand künstlich bewässert werden müssen, z.B. in ariden oder semiariden Gebieten. Durch den Einsatz der erfindungsgemäßen Substanzen läßt sich die Intensität der Bewässerung reduzieren und damit eine kostengünstigere Bewirtschaftung durhführen. Unter dem Einfluß von Wachstumsregulatoren kommt es zu einer besseren Ausnutzung des vorhandenen Wassers, weil u.a.

- die Öffnungsweite der Stomata reduziert wird
- eine dickere Epidermis und Cuticula ausgebildet werden
- die Durchwurzelung des Bodens verbessert wird
- das Mikroklima im Pflanzenbestand durch einen kompakteren Wuchs günstig beeinflußt wird.

Die erfindungsgemäß zu verwendenden Wirkstoffe der Formel I (A = Sauerstoff) können den Kulturpflanzen sowohl vom Samen her (als Saatgutbeizmittel) als auch über den Boden, d.h. durch die Wurzel sowie - besonders bevorzugt - durch Spritzung über das Blatt zugeführt werden.
Infolge der hohen Pflanzenverträglichkeit kann die Aufwandmenge stark variiert werden.

Bei der Saatgutbehandlung werden im allgemeinen Wirkstoffmengen von 0,001 bis 50 g je Kilogramm Saatgut, vorzugsweise 0,01 bis 10 g, benötigt.

Für die Blatt- und Bodenbehandlung sind im allgemeinen Gaben von 0,01 bis 10 kg/ha, bevorzugt 0,05 bis 3 kg/ha, als ausreichend zu betrachten.

Zur Bestimmung der wachstumsregulierenden Eigenschaft der Wirkstoffe wurden Testpflanzen auf ausreichend mit Nährstoffen versorgtem Kultursubstrat in Kunststoffgefäßen von ca. 12,5 cm Durchmesser angezogen.

Im Vorauflaufverfahren wurden die Wirkstoffe in wäßriger Aufbereitung am Tage der Einsaat auf das Saatbett gegossen.

Im Nachauflaufverfahren wurden die Wirkstoffe in wäßriger Aufbereitung auf die Pflanzen gesprüht. Die beobachtete wachstumsregulierende Wirkung wurde bei Versuchsende durch Wuchshöhenmessung belegt. Die so gewonnenen Meßwerte wurden zur Wuchshöhe der unbehandelten Pflanzen in Relation gesetzt. Als Vergleichssubstanz diente Chlorcholinchlorid.

Gleichlaufend zur Reduzierung des Längenwachstums stieg die Farbintensität der Blätter an. Der erhöhte Chlorophyllgehalt läßt eine ebenfalls erhöhte Photosyntheserate und damit eine erhöhte Ertragsbildung erwarten.

Die beispielhaft ausgewählten Wirkstoffe Nr. 310, 325 und 366 zeigen bei Nachauflaufanwendung deutliche wachstumsregulierende Eigenschaften.

Das erfindungsgemäße, das Wachstum regulierende Mittel kann in den genannten Anwendungsformen auch zusammen mit anderen Wirkstoffen vorliegen, wie Herbizide, Insektizide, Wachstumsregulatoren und Fungizide, oder auch mit Düngemitteln vermischt und ausgebracht werden. Beim Vermischen mit Wachstumsregulatormischungen treten auch synergistische Effekte auf, d.h. die Wirksamkeit des Kombinationsproduktes ist größer als die addierten Wirksamkeiten der Einzelkomponenten.

Fungizide die mit den neuen Wirkstoffen der Formel I (A=Sauerstoff) kombiniert werden können, sind beispielsweise:

Schwefel,
Dithiocarbamate und deren Derivate, wie
Ferridimethyldithiocarbamat,
Zinkdimethyldithiocarbamat,
Zinkethylenbisdithiocarbamat,
Manganethylenbisdithiocarbamat,
Mangan-Zink-ethylendiamin-bis-dithiocarbamat,
Tetramethylthiuramdisulfide,
Ammoniak-Komplex von Zink-(N,N-ethylen-bis-dithiocarbamat),
Ammoniak-Komplex von Zink-(N,N'-propylen-bis-dithiocarbamat),

Zink-(N,N'-propylen-bis-dithiocarbamat),
N,N'-Polypropylen-bis-(thiocarbamoyl)-disulfid;

Nitroderivate, wie
Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat;
5-Nitro-isophthalsäure-di-isopropylester

heterocyclische Substanzen, wie
2-Heptadecyl-2-imidazolin-acetat,
2,4-Dichlor-6-(o-chloranilino)-s-triazin,
O,O-Diethyl-phthalimidophosphonothioat,
5-Amino-1-[bis-(dimethylamino)-phosphinyl]-3-phenyl-1,2,4-triazol,
2,3-Dicyano-1,4-dithioanthrachinon,
2-Thio-1,3-dithio-(4,5-b)-chinoxalin,
1-(Butylcarbamoyl)-2-benzimidazol-carbaminsäuremethylester,
2-Methoxycarbonylamino-benzimidazol,
2-(Furyl-(2))-benzimidazol,
2-(Thiazolyl-(4))-benzimidazol,
N-(1,1,2,2-Tetrachlorethylthio)-tetrahydrophthalimid,
N-Trichlormethylthio-phthalimid,
N-Dichlorfluormethylthio-N',N'-dimethyl-N-phenyl-schwefelsäurediamid,
5-Ethoxy-3-trichlormethyl-1,2,3-thiadiazol,
2-Rhodanmethylthiobenzthiazol,

1,4-Dichlor-2,5-dimethoxybenzol,
4-(2-Chlorphenylhydrazono)-3-methyl-5-isoxazolon,

Pyridin-2-thio-1-oxid,
8-Hydroxychinolin bzw. dessen Kupfersalz,
2,3-Dihydro-5-carboxanilido-6-methyl-1,4-oxathiin,
2,3-Dihydro-5-carboxanilido-6-methyl-1,4-oxathiin-4,4-dioxid,
2-Methyl-5,6-dihydro-4H-pyran-3-carbonsäure-anilid,
2-Methyl-furan-3-carbonsäureanilid,
2,5-Dimethyl-furan-3-carbonsäureanilid,
2,4,5-Trimethyl-furan-3-carbonsäureanilid,
2,5-Dimethyl-furan-3-carbonsäurecyclohexylamid,
N-Cyclohexyl-N-methoxy-2,5-dimethyl-furan-3-carbonsäureamid,
2-Methyl-benzoesäure-anilid,
2-Iod-benzoesäure-anilid,
N-Formyl-N-morpholin-2,2,2-trichlorethylacetal,

Piperazin-1,4-diyl-bis-(1-(2,2,2-trichlor-ethyl)-formamid,
1-(3,4-Dichloranilino)-1-formylamino-2,2,2-trichlorethan ,
2,6-Dimethyl-N-tridecyl-morpholin bzw. dessen Salze,
2,6-Dimethyl-N-cyclodedecyl-morpholin bzw. dessen Salze,
N-[3-(p-tert.-Butylphenyl)-2-methylpropyl]-cis-2,6-dimethylmorpholin,
N-[3-(p-tert.-Butylphenyl)-2-methylpropyl]-piperidin,
1-[2-(2,4-Dichlorphenyl)-4-ethyl-1,3-dioxolan-2-yl-ethyl]-1H-1,2,4-triazol
1-[2-(2,4-Dichlorphenyl)-4-n-propyl-1,3-dioxolan-2-yl-ethyl]-1H-1,2,4-triazol,
N-(n-Propyl)-N-(2,4,6-trichlorphenoxyethyl)-N'-imidazolyl-harnstoff,
1-(4-Chlorphenoxy)-3,3-dimethyl-1-(1H-1,2,4-triazol-1-yl)-2-butanon,
1-(4-Chlorphenoxy)-3,3-dimethyl-1-(1H-1,2,4-triazol-1-yl)-2-butanol,
α-(2-Chlorphenyl)-α-(4-chlorphenyl)-5-pyrimidin-methanol,
5-Butyl-2-dimethylamino-4-hydroxy-6-methyl-pyrimidin,
Bis-(p-chlorphenyl)-3-pyridinmethanol,
1,2-Bis-(3-ethoxycarbonyl-2-thioureido)-benzol,
1,2-Bis-(3-methoxycarbonyl-2-thioureido)-benzol,

sowie verschiedene Fungizide, wie
Dodecylguanidinacetat,
3-[3-(3,5-Dimethyl-2-oxycyclohexyl)-2-hydroxyethyl]-glutarimid,
Hexachlorbenzol,
DL-Methyl-N-(2,6-dimethyl-phenyl)-N-furoyl(2)-alaninat,
DL-N-(2,6-Dimethyl-phenyl)-N-(2'-methoxyacetyl)-alanin-methylester,
N-(2,6-Dimethylphenyl)-N-chloracetyl-D,L-2-aminobutyrolacton,
DL-N-(2,6-Dimethylphenyl)-N-(phenylacetyl)-alaninmethylester,
5-Methyl-5-vinyl-3-(3,5-dichlorphenyl)-2,4-dioxo-1,3-oxazolidin,

3-(3,5-Dichlorphenyl)-5-methyl-5-methoxymethyl-1,3-oxazolidin-2,4-dion,
3-(3,5-Dichlorphenyl)-1-isopropylcarbamoylhydantoin,
N-(3,5-Dichlorphenyl)-1,2-dimethylcyclopropan-1,2-dicarbonsäureimid,
2-Cyano-[N-(ethylaminocarbonyl)-2-methoximino]-acetamid,
1-[2-(2,4-Dichlorphenyl)-pentyl]-1H-1,2,4-triazol,
2,4-Difluor-α-(1H-1,2,4-triazolyl-1-methyl)-benzhydrylalkohol.

**Patentansprüche**

1. Cyclohexenonderivate der Formel I

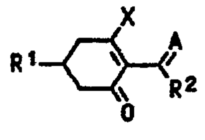

(I),

in der
$R^1$ einen 5-bis 7-gliedrigen Heterocyclus mit 1 bis 3 gleichen oder verschiedenen Heteroatomen oder Ringgliedern, ausgewählt aus der Gruppe, umfassend N, O, S, SO und $SO_2$, der gegebenenfalls 1 bis 3 Doppelbindungen und bis zu 3 Substituenten, ausgewählt aus der Gruppe, umfassend $C_1$-$C_6$-Alkyl, $C_2$-$C_6$-Alkenyl, $C_1$-$C_6$-Alkoxy, $C_1$-$C_6$-Alkylthio, $C_1$-$C_4$-Dialkylamino und $C_2$-$C_8$-Alkoxyalkyl enthält, 2-Ethylthiopropyl oder $C_1$-$C_6$-Alkoxycarbonyl,
$R^2$ $C_1$-$C_4$-Alkyl,
A Sauerstoff oder den Rest $NOR^3$, in dem $R^3$ für $C_1$-$C_4$-Alkyl, $C_3$- und $C_4$-Alkenyl, $C_3$- oder $C_4$-Alkinyl,

$C_2$-$C_4$-Halogenalkyl, $C_3$-$C_4$-Halogenalkenyl, $C_2$-$C_4$-Alkoxyalkyl steht, und
XHalogen, Amino, $C_1$-$C_4$-Alkylamino, $C_1$-$C_4$-Dialkylamino, Phenylamino, Pyrrolidino, Piperidino, Morpholino, Hexamethylenimino, $C_1$-$C_4$-Alkylthio, Phenylthio oder ein Azol, ausgewählt aus der Gruppe, umfassend Pyrrol, Pyrazol, Imidazol und 1,2,4-Triazol, das über ein Stickstoffatom an den Cyclohexanring gebunden ist, bedeuten.

2. Cyclohexanonderivat gemäß Anspruch 1, dadurch gekennzeichnet, daß
$R^1$ Tetrahydropyranyl oder Tetrahydrothiopyranyl,
$R^2$ Ethyl oder Propyl,
Aden Rest $NOR^3$,
$R^3$ Ethyl, Butenyl, Chlorallyl und
XChlor, Morpholino oder Methylthio bedeutet.

3. Verfahren zur Herstellung von Cyclohexenonderivaten der Formel I gemäß Anspruch 1, dadurch gekennzeichnet, daß man eine Verbindung der Formel II

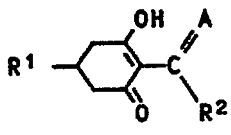 (II),

in der $R^1$, $R^2$ und A jeweils die in Anspruch 1 genannte Bedeutung besitzen, mit einem Chlorierungs- oder Bromierungsmittel, gegebenenfalls in Gegenwart einer Base, bei einer Temperatur von -10 bis +120°C umsetzt und gegebenenfalls die resultierende Chlor- oder Bromverbindung mit einem Nucleophil der Formel X-H, in der X mit Ausnahme von Chlor und Brom die in Anspruch1 genannte Bedeutung besitzt, gegebenenfalls in Gegenwart einer Base bei einer Temperatur von -10 bis +120°C umsetzt.

4. Herbizid, enthaltend einen inerten Zusatzstoff und ein Cyclohexenonderivat der Formel I

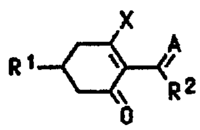 (I),

in der
$R^1$einen 5-bis 7-gliedrigen Heterocyclus mit 1 bis 3 gleichen oder verschiedenen Heteroatomen oder Ringgliedern, ausgewählt aus der Gruppe, umfassend N, O, S, SO und $SO_2$, der gegebenenfalls 1 bis 3 Doppelbindungen und bis zu 3Substituenten, ausgewählt aus der Gruppe, umfassend $C_1$-$C_6$-Alkyl, $C_2$-$C_6$-Alkenyl, $C_1$-$C_6$-Alkoxy, $C_1$-$C_6$-Alkylthio, $C_1$-$C_4$-Dialkylamino und $C_2$-$C_8$-Alkoxyalkyl enthält, 2-Ethylthiopropyl oder $C_1$-$C_6$-Alkoxycarbonyl,
$R^2$$C_1$-$C_4$-Alkyl,
ASauerstoff oder den Rest $NOR^3$, in dem $R^3$ für $C_1$-$C_4$-Alkyl, $C_3$- und $C_4$-Alkenyl, $C_3$- oder $C_4$-Alkinyl, $C_2$-$C_4$-Halogenalkyl, $C_3$-$C_4$-Halogenalkenyl, $C_2$-$C_4$-Alkoxyalkyl steht, und
XHalogen, Amino, $C_1$-$C_4$-Alkylamino, $C_1$-$C_4$-Dialkylamino, Phenylamino, Pyrrolidino, Piperidino, Morpholino, Hexamethylenimino, $C_1$-$C_4$-Alkylthio, Phenylthio oder ein Azol, ausgewählt aus der Gruppe, umfassend Pyrrol, Pyrazol, Imidazol und 1,2,4-Triazol, das über ein Stickstoffatom an den Cyclohexanring gebunden ist, bedeuten.

5. Verfahren zur Bekämpfung von unerwünschtem Pflanzenwachstum, dadurch gekennzeichnet, daß man die unerwünschten Pflanzen und/oder die von unerwünschtem Pflanzenwachstum freizuhaltende Fläche mit einer herbizid wirksamen Menge eines Cylohexenonderivats der Formel I

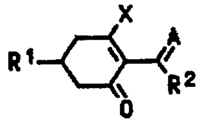 (I),

in der
$R^1$einen 5- bis 7-gliedrigen Heterocyclus mit 1 bis 3 gleichen oder verschiedenen Heteroatomen oder Ringgliedern, ausgewählt aus der Gruppe, umfassend N, O, S, SO und $SO_2$, der gegebenenfalls 1 bis 3 Doppelbindungen und bis zu 3 Substituenten, ausgewählt aus der Gruppe, umfassend $C_1$-$C_6$-Alkyl, $C_2$-$C_6$-Alkenyl, $C_1$-$C_6$-Alkoxy, $C_1$-$C_6$-Alkylthio, $C_1$-$C_4$-Dialkylamino und $C_2$-$C_8$-Alkoxyalkyl enthält, 2-Ethylthiopropyl oder $C_1$-$C_6$-Alkoxycarbonyl,
$R^2$$C_1$-$C_4$-Alkyl,
ASauerstoff oder den Rest $NOR^3$, in dem $R^3$ für $C_1$-$C_4$-Alkyl, $C_3$- und $C_4$-Alkenyl, $C_3$- oder $C_4$-Alkinyl, $C_2$-$C_4$-Halogenalkyl, $C_3$-$C_4$-Halogenalkenyl, $C_2$-$C_4$-Alkoxyalkyl steht, und
XHalogen, Amino, $C_1$-$C_4$-Alkylamino, $C_1$-$C_4$-Dialkylamino, Phenylamino, Pyrrolidino, Piperidino, Morpholino, Hexamethylenimino, $C_1$-$C_4$-Alkylthio, Phenylthio oder ein Azol, ausgewählt aus der Gruppe, umfassend Pyrrol, Pyrazol, Imidazol und 1,2,4-Triazol, das über ein Stickstoffatom an den Cyclohexanring gebunden ist, bedeuten, enthalten.

6. Mittel zur Regulierung des Pflanzenwachstums, enthaltend einen inerten Zusatzstoff und ein Cyclohexenonderivat der Formel I

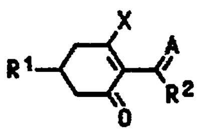

(I),

in der

$R^1$einen 5- bis 7-gliedrigen Heterocyclus mit 1 bis 3 gleichen oder verschiedenen Heteroatomen oder Ringgliedern, ausgewählt aus der Gruppe, umfassend N, O, S, SO und $SO_2$, der gegebenenfalls 1 bis 3 Doppelbindungen und bis zu 3 Substituenten, ausgewählt aus der Gruppe, umfassend $C_1$-$C_6$-Alkyl, $C_2$-$C_6$-Alkenyl, $C_1$-$C_6$-Alkoxy, $C_1$-$C_6$-Alkylthio, $C_1$-$C_4$-Dialkylamino und $C_2$-$C_8$-Alkoxyalkyl enthält, 2-Ethylthiopropyl oder $C_1$-$C_6$-Alkoxycarbonyl,

$R^2$$C_1$-$C_4$-Alkyl,

ASauerstoff und

XHalogen, Amino, $C_1$-$C_4$-Alkylamino, $C_1$-$C_4$-Dialkylamino, Phenylamino, Pyrrolidino, Piperidino, Morpholino, Hexamethylenimino, $C_1$-$C_4$-Alkylthio, Phenylthio oder ein Azol, ausgewählt aus der Gruppe, umfassend Pyrrol, Pyrazol, Imidazol und 1,2,4-Triazol, das über ein Stickstoffatom an den Cyclohexanring gebunden ist, bedeuten.

7. Verfahren zur Regulierung des Pflanzenwachstums, dadurch gekennzeichnet, daß man eine wirksame Menge eines Cyclohexenonderivats der Formel I

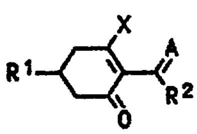

(I),

in der

$R^1$einen 5- bis 7-gliedrigen Heterocyclus mit 1 bis 3 gleichen oder verschiedenen Heteroatomen oder Ringgliedern, ausgewählt aus der Gruppe, umfassend N, O, S, SO und $SO_2$, der gegebenenfalls 1 bis 3 Doppelbindungen und bis zu 3 Substituenten, ausgewählt aus der Gruppe, umfassend $C_1$-$C_6$-Alkyl, $C_2$-$C_6$-Alkenyl, $C_1$-$C_6$-Alkoxy, $C_1$-$C_6$-Alkylthio, $C_1$-$C_4$-Dialkylamino und $C_2$-$C_8$-Alkoxyalkyl enthält, 2-Ethylthiopropyl oder $C_1$-$C_6$-Alkoxycarbonyl,

$R^2$$C_1$-$C_4$-Alkyl,

ASauerstoff und

XHalogen, Amino, $C_1$-$C_4$-Alkylamino, $C_1$-$C_4$-Dialkylamino, Phenylamino, Pyrrolidino, Piperidino, Morpholino, Hexamethylenimino, $C_1$-$C_4$-Alkylthio, Phenylthio oder ein Azol, ausgewählt aus der Gruppe, umfassend Pyrrol, Pyrazol, Imidazol und 1,2,4-Triazol, das über ein Stickstoffatom an den Cyclohexanring gebunden ist, bedeuten,

auf Pflanzen und/oder deren Lebensraum einwirken läßt.

## Claims

Cyclohexenone derivatives of the formula

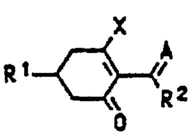

(I)

where

$R^1$ is a 5-, 6- or 7-membered heterocycle of 1, 2 or 3 identical or different hetero atoms or ring members selected from the group consisting of N, O, S, SO and $SO_2$, which may contain 1, 2 or 3 double bonds and up to 3 substituents selected from the group consisting of $C_1$–$C_6$-alkyl, $C_2$–$C_6$-alkenyl, $C_1$–$C_6$-alkoxy, $C_1$–$C_6$-alkylthio, $C_1$–$C_4$-dialkylamino and $C_2$–$C_8$-alkoxyalkyl, or is 2-ethylthiopropyl or $C_1$–$C_6$-alkoxycarbonyl,

$R^2$ is $C_1$–$C_4$-alkyl,

A is oxygen or $NOR^3$, where $R^3$ is $C_1$–$C_4$-alkyl, $C_3$–$C_4$-alkenyl, $C_3$-alkynyl, $C_4$-alkynyl, $C_2$–$C_4$-haloalkyl, $C_3$–$C_4$-haloalkenyl or $C_2$–$C_4$-alkoxyalkyl, and

X is halogen, amino, $C_1$–$C_4$-alkylamino, $C_1$–$C_4$-dialkylamino, phenylamino, pyrrolidino, piperidino, morpholino, hexamethyleneimino, $C_1$–$C_4$-alkylthio, phenylthio or an azole selected from the group consisting

of pyrrole, pyrazole, imidazole and 1,2,4-triazole, which is bonded to the cyclohexane ring via a nitrogen atom.

2. A cyclohexanone (sic) derivative as claimed in claim 1, where
$R^1$ is tetrahydropyranyl or tetrahydrothiopyranyl,
$R^2$ is ethyl or propyl,
A is $NOR^3$,
$R^3$ is ethyl, butenyl or chloroallyl, and
X is chlorine, morpholino or methylthio.

3. A process for preparing a cyclohexenone derivative of the formula I as claimed in claim 1, which comprises reacting a compound of the formula II

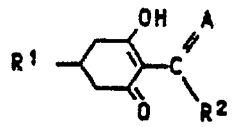

(II)

where $R^1$, $R^2$ and A have the meanings stated in claim 1, with a chlorinating or brominating agent at from −10 to +120°C in the presence or absence of a base and, if appropriate, reacting the resulting chlorine or bromine compound with a nucleophilic of the formula X–H, where X has the meanings stated in claim 1 other than chlorine and bromine, at from −10 to +120°C in the presence or absence of a base.

4. A herbicide containing an inert additive and a cyclohexenone derivative of the formula I

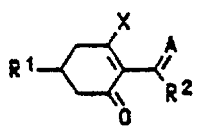

(I)

where
$R^1$ is a 5-, 6- or 7-membered heterocycle of 1, 2 or 3 identical or different hetero atoms or ring members selected from the group consisting of N, O, S, SO and $SO_2$, which may contain 1, 2 or 3 double bonds and up to 3 substituents selected from the group consisting of $C_1$–$C_6$-alkyl, $C_2$–$C_6$-alkenyl, $C_1$–$C_6$-alkoxy, $C_1$–$C_6$-alkylthio, $C_1$–$C_4$-dialkylamino and $C_2$–$C_8$-alkoxyallyl, or is 2-ethylthiopropyl or $C_1$–$C_6$-alkoxycarbonyl,
$R^2$ is $C_1$–$C_4$-alkyl,
A is oxygen or $NOR^3$, where $R^3$ is $C_1$–$C_4$-alkyl, $C_3$–$C_4$-alkyl, $C_3$–$C_4$-alkenyl, $C_3$-alkynyl, $C_4$-alkynyl, $C_2$–$C_4$-haloalkyl, $C_3$–$C_4$-haloalkenyl or $C_2$–$C_4$-alkoxyalkyl, and
X is halogen, amino, $C_1$–$C_4$-alkylamino, $C_1$–$C_4$-dialkylamino, phenylamino, pyrrolidino, piperidino, morpholino, hexamethyleneimino, $C_1$–$C_4$-alkylthio, phenylthio or an azole selected from the group consisting of pyrrole, pyrazole, imidazole and 1,2,4-triazole, which is bonded to the cyclohexane ring via a nitrogen atom.

5. A process for combating undesirable plant growth, wherein the undesirable plants and/or the area to be kept free from undesirable plant growth are contain (sic) with a herbicidally effective amount of a cyclohexenone derivative of the formula I

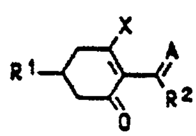

(I)

where
$R^1$ is a 5-, 6- or 7-membered heterocycle of 1, 2 or 3 identical or different hetero atoms or ring members selected from the group consisting of N, O, S, SO and $SO_2$, which may contain 1, 2 or 3 double bonds and up to 3 substituents selected from the group consisting of $C_1$–$C_6$-alkyl, $C_2$–$C_6$-alkenyl, $C_1$–$C_6$-alkoxy, $C_1$–$C_6$-alkylthio, $C_1$–$C_4$-dialkylamino and $C_2$–$C_8$-alkoxyalkyl, or is 2-ethylthiopropyl or $C_1$–$C_6$-alkoxycarbonyl,
$R^2$ is $C_1$–$C_4$-alkyl,
A is oxygen or $NOR^3$, where $R^3$ is $C_1$–$C_4$-alkyl, $C_3$–$C_4$-alkenyl, $C_3$-alkynyl, $C_4$-alkynyl, $C_2$–$C_4$-haloalkyl, $C_3$–$C_4$-haloalkenyl or $C_2$–$C_4$-alkoxyalkyl, and
X is halogen, amino, $C_1$–$C_4$-alkylamino, $C_1$–$C_4$-dialkyl-amino, phenylamino, pyrrolidino, piperidino, morpholino, hexamethyleneimino, $C_1$–$C_4$-alkylthio, phenylthio or an azole selected from the group consisting of pyrrole, pyrazole, imidazole and 1,2,4-triazole, which is bonded to the cyclohexane ring via a nitrogen atom.

6. An agent for regulating plant growth, containing an inert additive and a cyclohexenone derivative of the formula I

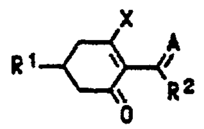

(I)

where

$R^1$ is a 5-, 6- or 7-membered heterocycle of 1, 2 or 3 identical or different hetero atoms or ring members selected from the group consisting of N, O, S, SO and $SO_2$, which may contain 1, 2 or 3 double bonds and up to 3 substituents selected from the group consisting of $C_1$–$C_6$-alkyl, $C_2$–$C_6$-alkenyl, $C_1$–$C_6$-alkoxy, $C_1$–$C_6$-alkylthio, $C_1$–$C_4$-dialkylamino and $C_2$–$C_8$-alkoxyalkyl, or is 2-ethylthiopropyl or $C_1$–$C_6$-alkoxycarbonyl,

$R^2$ is $C_1$–$C_4$-alkyl,

A is oxygen, and

X is halogen, amino, $C_1$–$C_4$-alkylamino, $C_1$–$C_4$-dialkylamino, phenylamino, pyrrolidino, piperidino, morpholino, hexamethyleneimino, $C_1$–$C_4$-alkylthio, phenylthio or an azole selected from the group consisting of pyrrole, pyrazole, imidazole and 1,2,4-triazole, which is bonded to the cyclohexane ring via a nitrogen atom.

7. A process for regulating plant growth, wherein an effective amount of a cyclohexenone derivative of the formula I

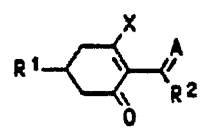

(I)

where

$R^1$ is a 5-, 6- or 7-membered heterocycle of 1, 2 or 3 identical or different hetero atoms or ring members selected from the group consisting of N, O, S, SO and $SO_2$, which may contain 1, 2 or 3 double bonds and up to 3 substituents selected from the group consisting of $C_1$–$C_6$-alkyl, $C_2$–$C_6$-alkenyl, $C_1$–$C_6$-alkoxy, $C_1$–$C_6$-alkylthio, $C_1$–$C_4$-dialkylamino and $C_2$–$C_8$-alkoxyalkyl, or is 2-ethylthiopropyl or $C_1$–$C_6$-alkoxycarbonyl,

$R^2$ is $C_1$–$C_4$-alkyl,

A is oxygen, and

X is halogen, amino, $C_1$–$C_4$-alkylamino, $C_1$–$C_4$-dialkylamino, phenylamino, pyrrolidino, piperidino, morpholino, hexamethyleneimino, $C_1$–$C_4$-alkylthio, phenylthio or an azole selected from the group consisting of pyrrole, pyrazole, imidazole and 1,2,4-triazole, which is bonded to the cyclohexane ring via a nitrogen atom, is allowed to act on plants and/or their habitat.

## Revendications

1. Dérivés de cyclohexènone de formule I

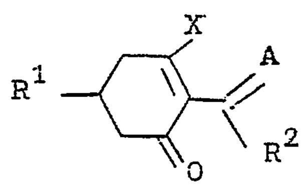

(I)

dans laquelle

$R^1$ représente un hétérocycle de 5 à 7 chaînons, à 1 à 3 hétéroatomes ou termes cycliques identiques ou différents, choisis dans le groupe comprenant N, O, S, SO et $SO_2$, qui contient éventuellement 1 à 3 doubles liaisons et jusqu'à 3 substituants, choisis dans le groupe comprenant alkyle en $C_1$–$C_6$ alcényle en $C_2$–$C_6$, alcoxy en $C_1$–$C_6$, alkylthio en $C_1$–$C_6$, dialkylamino en $C_1$–$C_4$ et alcoxyalkyle en $C_2$–$C_8$, 2-éthylthiopropyle ou alcoxycarbonyle en $C_1$–$C_6$

$R^2$ représente alkyle en $C_1$–$C_4$

A représente oxygène ou le reste $NOR^3$, dans lequel $R^3$ est mis pour alkyle en $C_1$–$C_4$, alcényle en $C_3$ et $C_4$, alkinyle en $C_3$ ou $C_4$, halogénoalkyle en $C_2$–$C_4$, halogénoalcényle en $C_3$–$C_4$, alcoxyalkyle en $C_2$–$C_4$ et

X représente halogène, amino, alkylamino en $C_1$–$C_4$, dialkylamino en $C_1$–$C_4$, phénylamino, pyrrolidino, pipéridino, morpholino, hexaméthylènimino, alkylthio en $C_1$–$C_4$, phénylthio ou un azole choisi dans le groupe comprenant pyrrole, pyrazole, imidazole et 1,2,4-triazole, qui est relié au noyau cyclohexane par un atome d'azote.

2. Dérivé de cyclohexenone selon la revendication 1, caractérisé par le fait que
$R^1$ représente tétrahydropyranyle ou tétrahydrothiopyranyle,
$R^2$ éthyle ou propyle
A le reste $NOR^3$,
$R^3$ éthyle, butényle, chlorallyle et
X chlore, morpholino ou méthylthio.

3. Procédé de préparation de dérivés de cyclohexenone de formule I selon la revendication 1, caractérisé par le fait que l'on fait réagir un composé de formule II

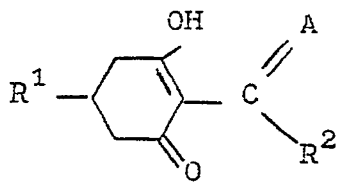

(II)

dans laquelle
$R^1$, $R^2$ et A ont chacun la signification donnée dans la revendication 1, avec un agent de chloration ou bromation, éventuellement en présence d'une base, à une température de –10 à +120°C, et éventuellement on fait réagir en présence d'une base, à une température de –10 à +120°C le composé chloré ou bromé résultant avec un nucléophile de formule X – H, dans laquelle X a la signification donnée dans la revendication 1, à l'exception de chlore et brome, en présence d'une base, à une température de –10 à +120°C.

4. Herbicide, contenant un additif inerte et un dérivé de cyclohexènone de formule I

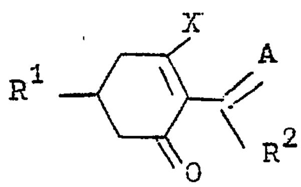

(I)

dans laquelle
$R^1$ représente un hétérocycle de 5 à 7 chaînons, à 1 à 3 hétéroatomes ou termes cycliques identiques ou différents, choisis dans le groupe comprenant N, O, S, SO et $SO_2$, qui contient éventuellement 1 à 3 doubles liaisons et jusqu'à 3 substituants, choisis dans le groupe comprenant alkyle en $C_1$–$C_6$ alcényle en $C_2$–$C_6$, alcoxy en $C_1$–$C_6$, alkylthio en $C_1$–$C_6$, dialkylamino en $C_1$–$C_4$ et alcoxyalkyle en $C_2$–$C_8$, 2-éthylthiopropyle ou alcoxycarbonyle en $C_1$–$C_6$
$R^2$ représente alkyle en $C_1$–$C_4$
A représente oxygène ou le reste $NOR^3$, dans lequel $R^3$ est mis pour alkyle en $C_1$–$C_4$, alcényle en $C_3$ et $C_4$, alkinyle en $C_3$ ou $C_4$, halogénoalkyle en $C_2$–$C_4$, halogénoalcényle en $C_3$–$C_4$, alcoxyalkyle en $C_2$–$C_4$ et
X représente halogène, amino, alkylamino en $C_1$–$C_4$, dialkylamino en $C_1$–$C_4$, phénylamino, pyrrolidino, pipéridino, morpholino, hexaméthylènimino, alkylthio en $C_1$–$C_4$, phénylthio ou un azole choisi dans le groupe comprenant pyrrole, pyrazole, imidazole et 1,2,4-triazole, qui est relié au noyau cyclohexane par un atome d'azote.

5. Procédé de lutte contre la croissance des plantes, caractérisé par le fait que l'on traite les plantes indésirables, et/ou les surfaces à maintenir exemptes de croissance de plantes indésirables, avec une quantité efficace du point de vue herbicide d'un dérivé de cyclohexénone de formule I

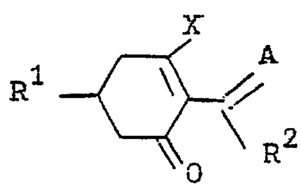

(I)

dans laquelle
$R^1$ représente un hétérocycle de 5 à 7 chaînons, à 1 à 3 hétéroatomes ou termes cycliques identiques ou différents, choisis dans le groupe comprenant N, O, S, SO et $SO_2$, qui contient éventuellement 1 à 3 doubles liaisons et jusqu'à 3 substituants, choisis dans le groupe comprenant alkyle en $C_1$–$C_6$ alcényle en $C_2$–$C_6$, alcoxy en $C_1$–$C_6$, alkylthio en $C_1$–$C_6$, dialkylamino en $C_1$–$C_4$ et alcoxyalkyle en $C_2$–$C_8$, 2-éthylthiopropyle ou alcoxycarbonyle en $C_1$–$C_6$
$R^2$ représente alkyle en $C_1$–$C_4$

A représente oxygène ou le reste $NOR^3$, dans lequel $R^3$ est mis pour alkyle en $C_1$–$C_4$, alcényle en $C_3$ et $C_4$, alkinyle en $C_3$ ou $C_4$, halogénoalkyle en $C_2$–$C_4$, halogénoalcényle en $C_3$–$C_4$, alcoxyalkyle en $C_2$–$C_4$ et
X représente halogène, amino, alkylamino en $C_1$–$C_4$, dialkylamino en $C_1$–$C_4$, phénylamino, pyrrolidino, pipéridino, morpholino, hexaméthylènimino, alkylthio en $C_1$–$C_4$, phénylthio ou un azole choisi dans le groupe comprenant pyrrole, pyrazole, imidazole et 1,2,4-triazole, qui est relié au noyau cyclohexane par un atome d'azote.

6. Moyen de régulation de la croissance des plantes, contenant un additif inerte et un dérivé de cyclohenenone de formule I

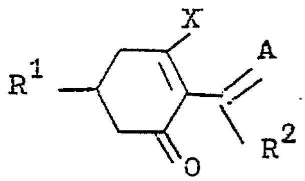

(I)

dans laquelle
$R^1$ représente un hétérocycle de 5 à 7 chaînons, à 1 à 3 hétéroatomes ou termes cycliques identiques ou différents, choisis dans le groupe comprenant N, O, S, SO et $SO_2$, qui contient éventuellement 1 à 3 doubles liaisons et jusqu'à 3 substituants, choisis dans le groupe comprenant alkyle en $C_1$–$C_6$ alcényle en $C_2$–$C_6$, alcoxy en $C_1$–$C_6$, alkylthio en $C_1$–$C_6$, dialkylamino en $C_1$–$C_4$ et alcoxyalkyle en $C_2$–$C_8$, 2-éthylthiopropyle ou alcoxycarbonyle en $C_1$–$C_6$
$R^2$ représente alkyle en $C_1$–$C_4$
A représente oxygène ou le reste $NOR^3$, dans lequel $R^3$ est mis pour alkyle en $C_1$–$C_4$, alcényle en $C_3$ et $C_4$, alkinyle en $C_3$ ou $C_4$, halogénoalkyle en $C_2$–$C_4$, halogénoalcényle en $C_3$–$C_4$, alcoxyalkyle en $C_2$–$C_4$ et
X représente halogène, amino, alkylamino en $C_1$–$C_4$, dialkylamino en $C_1$–$C_4$, phénylamino, pyrrolidino, pipéridino, morpholino, hexaméthylènimino, alkylthio en $C_1$–$C_4$, phénylthio ou un azole choisi dans le groupe comprenant pyrrole, pyrazole, imidazole et 1,2,4-triazole, qui est relié au noyau cyclohexane par un atome d'azote.

7. Procédé de régulation de la croissance des plantes, caractérisé par le fait que l'on fait agir sur les plantes et/ou leur biotope, une quantité efficace d'un dérivé de cyclohexenone de formule I

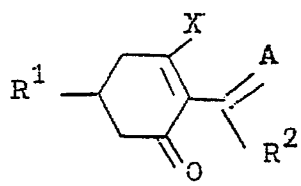

(I)

dans laquelle
$R^1$ représente un hétérocycle de 5 à 7 chaînons, à 1 à 3 hétéroatomes ou termes cycliques identiques ou différents, choisis dans le groupe comprenant N, O, S, SO et $SO_2$, qui contient éventuellement 1 à 3 doubles liaisons et jusqu'à 3 substituants, choisis dans le groupe comprenant alkyle en $C_1$–$C_6$ alcényle en $C_2$–$C_6$, alcoxy en $C_1$–$C_6$, alkylthio en $C_1$–$C_6$, dialkylamino en $C_1$–$C_4$ et alcoxyalkyle en $C_2$–$C_8$, 2-éthylthiopropyle ou alcoxycarbonyle en $C_1$–$C_6$
$R^2$ représente alkyle en $C_1$–$C_4$
A représente oxygène ou le reste $NOR^3$, dans lequel $R^3$ est mis pour alkyle en $C_1$–$C_4$, alcényle en $C_3$ et $C_4$, alkinyle en $C_3$ ou $C_4$, halogénoalkyle en $C_2$–$C_4$, halogénoalcényle en $C_3$–$C_4$, alcoxyalkyle en $C_2$–$C_4$ et
X représente halogène, amino, alkylamino en $C_1$–$C_4$, dialkylamino en $C_1$–$C_4$, phénylamino, pyrrolidino, pipéridino, morpholino, hexaméthylènimino, alkylthio en $C_1$–$C_4$, phénylthio ou un azole choisi dans le groupe comprenant pyrrole, pyrazole, imidazole et 1,2,4-triazole, qui est relié au noyau cyclohexane par un atome d'azote.